(19) Europäisches Patentamt
European Patent Office
Office européen des brevets

(11) **EP 1 664 746 B1**

(12) **EUROPEAN PATENT SPECIFICATION**

(45) Date of publication and mention
of the grant of the patent:
**18.12.2013 Bulletin 2013/51**

(51) Int Cl.:
*G01N 33/18* (2006.01)     *A61K 31/353* (2006.01)
*A61K 31/355* (2006.01)     *A61K 31/7048* (2006.01)
*A61K 36/24* (2006.01)     *A61K 36/82* (2006.01)
*A61K 36/87* (2006.01)

(21) Application number: **04782194.7**

(22) Date of filing: **25.08.2004**

(86) International application number:
**PCT/US2004/027644**

(87) International publication number:
**WO 2005/022116 (10.03.2005 Gazette 2005/10)**

(54) **Antioxidant sensor and method**

Antioxidantiensensor und Verfahren

Capteur d'antioxydants et procédé

(84) Designated Contracting States:
**AT BE BG CH CY CZ DE DK EE ES FI FR GB GR
HU IE IT LI LU MC NL PL PT RO SE SI SK TR**

(30) Priority: **26.08.2003 US 648047**

(43) Date of publication of application:
**07.06.2006 Bulletin 2006/23**

(60) Divisional application:
**10159748.2 / 2 218 453**

(73) Proprietor: **Mannatech, Inc.
Coppell, TX 75019 (US)**

(72) Inventors:
• **MCANALLEY, Bill, H.
Grand Prairie
TX 75052 (US)**
• **VENNUM, Eileen, P.
Grand Prairie
TX 75050 (US)**
• **MCANALLEY, Shayne, A.
Grand Prairie
TX 75052 (US)**
• **KOEPKE, Michael, C.
Houston
TX 77030 (US)**

(74) Representative: **HOFFMANN EITLE
Patent- und Rechtsanwälte
Arabellastrasse 4
81925 München (DE)**

(56) References cited:
**US-A- 5 427 951    US-A- 6 114 177
US-A- 6 114 177    US-B1- 6 177 260
US-B1- 6 365 622    US-B1- 6 429 021
US-B1- 6 562 869    US-B1- 6 607 919**

• **ROGINSKY VITALY: "Chain-breaking antioxidant
activity of natural polyphenols as determined
during the chain oxidation of methyl linoleate in
Triton X-100 micelles." ARCHIVES OF
BIOCHEMISTRY AND BIOPHYSICS, vol. 414, no.
2, 15 June 2003 (2003-06-15), pages 261-270,
XP002507143 ISSN: 0003-9861**
• **BERGMAN M ET AL: "The antioxidant activity of
aqueous spinach extract: chemical identification
of active fractions" PHYTOCHEMISTRY,
PERGAMON PRESS, GB, vol. 58, no. 1, 1
September 2001 (2001-09-01), pages 143-152,
XP004301205 ISSN: 0031-9422**
• **UGWU S O ET AL: "SYSTEMATIC SCREENING
OF ANTIOXIDANTS FOR MAXIMUM
PROTECTION AGAINST OXIDATION: AN
OXYGEN POLAROGRAPH STUDY" PDA
JOURNAL OF PHARMACEUTICAL SCIENCE AND
TECHNOLOGY, BETHESDA, MD, US, vol. 53, no.
5, 1 September 1999 (1999-09-01), pages 252-259,
XP009037912 ISSN: 1079-7440**

- HUANG D ET AL: "Development and validation of oxygen radical absorbance capacity assay for lipophilic antioxidants using randomly methylated beta-cyclodextrin as the solubility enhancer" JOURNAL OF AGRICULTURAL AND FOOD CHEMISTRY, AMERICAN CHEMICAL SOCIETY. WASHINGTON, US, vol. 50, no. 7, 8 February 2002 (2002-02-08), pages 1815-1821, XP002375230 ISSN: 0021-8561
- Pamela Pedrielli ET AL: "Antioxidant Synergy and Regeneration Effect of Quercetin, (-)-Epicatechin, and (+)-Catechin on [alpha]-Tocopherol in Homogeneous Solutions of Peroxidating Methyl Linoleate", Journal of Agricultural and Food Chemistry, vol. 50, no. 24, 1 November 2002 (2002-11-01), pages 7138-7144, XP055051865, ISSN: 0021-8561, DOI: 10.1021/jf020437l
- COATES BARCLAY L R ET AL: "Autoxidation in micelles. Synergism of vitamin C with lipid-soluble vitamin E and water-soluble Trolox", CANADIAN JOURNAL OF CHEMISTRY, NRC RESEARCH PRESS, CA, vol. 63, no. 2, 1 January 1985 (1985-01-01), pages 366-374, XP008087450, ISSN: 0008-4042, DOI: 10.1139/V85-062

**Description**

FIELD OF THE INVENTION

The present invention relates in general to the field of antioxidant sensors, and more particularly, to antioxidant sensors and methods that directly measure both hydrophilic and hydrophobic anti-oxidants.

BACKGROUND OF THE INVENTION

This application claims priority to U.S. Patent Application Serial No. 10/648,047, filed August 26, 2003. Without limiting the scope of the invention, its background is described in connection with antioxidant sensor technology and methods for detection.

Biological systems have developed antioxidant systems to combat the effects of radicals and other pro-oxidative species. An antioxidant is any substance that significantly delays or prevents oxidation of an oxidizable substrate when present at low concentrations compared to that of the oxidizable substrate. There are enzymes that are antioxidants, such as superoxide dismutase and catalase, which are coded for by many organisms. Substances such as vitamin C and plant phenols are antioxidants introduced through the diet into biological systems. It has been proposed that naturally occurring levels of these substances are not adequately produced in the body or ingested in the normal diet. The normal diet often does not provide enough antioxidants because it is deficient in fruits and vegetables and/or the fruits and vegetables that are in the diet are depleted of their antioxidants due to modem-day processing. The normal diet could be improved, however, today's lifestyles and poor composition of western foods, make supplementation the most practical way to deliver the anti-oxidants required by the body. To supplement the normal diet, it is necessary to determine the antioxidant capacities of the components included in the supplements.

Several laboratory methods have been developed to determine the ability of a substance to quench a free radical or to determine its antioxidant capacity; each of these assays are described in detail hereinbelow. Briefly, these assays include: TEAC, $^{19}$F-NMR, TRAP, modified TRAP, FRAP, fluorescence-based methods, phosphomolybdenum complex detection, and the ORAC, all of which are proposed to measure some aspect of a substance's ability to quench free radical species. The following is a brief description of each method, including inherent advantages and disadvantages.

TEAC assay. The Trolox® Equivalent Antioxidant Capacity (TEAC) assay is based on the observation that when 2,2'-azinobis-(3-ethylbenzothiazoline-6-sulphonic acid) (ABTS) is incubated in the presence of a peroxidase and hydrogen peroxide or in the presence of hydroxyl, peroxyl, alkoxyl, and inorganic radicals, the slightly more stable $ABTS^{\bullet+}$ radical cation is generated. From the time the ABTS, metmyoglobin, buffer, and hydrogen peroxide are added together, the absorbance of infra red radiation is measured at a wavelength of 734 nm with respect to time. As the $ABTS^{\bullet+}$ radical cation begins to form, the absorbance increases. When antioxidants are added before the addition of hydrogen peroxide, the antioxidants scavenge the radicals formed by the hydrogen peroxide, delaying the formation of the $ABTS^{\bullet+}$ radical cation, thus inducing an increase in the percentage of inhibition of the absorbance. The unit of measure in this assay is the TEAC, which is the concentration (mmol/l) of Trolox® with the equivalent antioxidant capacity to a 1.0 mmol/l solution of the substance being tested.

The TEAC assay detects the antioxidant capacity of water-soluble drugs or drugs that can be solubilized. Also, due to the ability of the TEAC assay to detect the contribution of other components, the TEAC assay can be used to measure the antioxidant capacity of the system. The TEAC method can be used for pharmacological and nutritional studies. Use of the TEAC method is limited because peroxidases present in the sample yield a higher absorbance value and the chemical tested can also absorb at 734 nm. Therefore, the TEAC method cannot guarantee the specificity of the sample antioxidant to directly quench free radicals because of direct interactions between the antioxidant sample and the reagents due to the relatively low concentration of the $H_2O_2$. The TEAC method is also affected by dilution of the sample, yielding an increase in TEAC values at lower concentrations of the sample.

$^{19}$F-NMR Assay. Another method uses $^{19}$F-NMR (nuclear magnetic resonance), in which aromatic amines fluorinated with $^{19}$F-NMR is detected. The aromatic amines react rapidly with hydroxyl radicals to form a mixture of hydroxylated products. The fluorinated detector, N-(4-hydroxyphenyl)-trifluoroacetamide, is broken down by hydroxyl radical attack to yield $CF_3CONH_2$, trifluoroacetamide (TFAM), along with other products. TFAM is used to determine the ability of a substance to protect the fluorinated detector from hydroxyl radical attack. If the sample is good at protecting the fluorinated detector from free radical attack, then the area under the TFAM peak will be smaller than if the substance is poor at protecting the fluorinated detector. The reagents are mixed together, and a measurement is taken using NMR. The area of the peak is measured and then normalized against the total concentration of the fluorine-containing species. The $^{19}$F-NMR method is a simple method that measures the antioxidant properties of low molecular weight biomolecules, however, the indicator only appears if hydroxyl radicals are involved, radioactive fluorine us used in the method and the NMR equipment is extremely expensive to purchase and operate.

TRAP Assay. Aqueous dispersions of oxidizable organic compounds are readily and reproducibly initiated at

a constant rate, $R_i$, by peroxidation using the water-soluble azo compound 2,2'-azo-bis-(2-amidipropane hydrochloride)(ABAP), which serves as the basis for the Total Radical-Trapping Antioxidant Parameter (TRAP) method. The TRAP method detects the length of time that oxygen uptake by peroxidizable plasma is inhibited with an oxygen probe, and this value is referred to as the TRAP. Trolox® is used as an anti-oxidant control in this method during a second induction period after the natural antioxidants have been depleted. The second induction period is used to calculate an $R_i$ value, which is used to calculate the TRAP value. The TRAP value is reported as the number of moles of peroxyl radicals trapped per liter of fluid.

Unfortunately, the duration of time is the only thing measured in the TRAP method, limiting its usefulness for high throughput analysis. The time taken to prevent maximum oxygen uptake cannot be measured easily and precisely, the total radical trapping capability per mole of some antioxidants is dependent on their initial concentration; and the actual extent of inhibition is not measured. The TRAP method is time consuming because, with only one reaction vessel, there can only be one sample tested at a time. While the TRAP method is more specific than the TEAC method because the TRAP method requires high levels of dilution of plasma to produce the required lag phase, and the process to accomplish this shortens the lipid chain length necessary for a rapid chain reaction. It was proposed that by adding linoleic acid, the shortening of chain length could be compensated for, but upon further study it was shown that the addition of linoleic acid introduced other sources of error (Ghiselli A; Serafini M; Maiani G; Azzini E; Ferro-Luzzi A., A fluorescence-based method for measuring total plasma antioxidant capability. Free Radic. Biol. Med. 1995 Jan; 18: 29-36). In the TRAP method assay system, the total peroxyl radical-trapping ability of some antioxidants such as vitamin C was dependent on the initial concentration.

Modified TRAP Assay. A modified TRAP assay corrects for the interferences from plasma proteins or sample dilution (Ghiselli, et al., supra). The modified TRAP method indirectly measures the effect of peroxyl radical attack produced by ABAP on the fluorescent properties of the protein $\beta$-phycoerythrin ($\beta$-PE) and the ability of plasma to protect $\beta$-PE. Protection is accomplished by precipitating the protein out of plasma with ammonium sulfate and ultracentrifugation. The modified TRAP assay is performed by adding the reagents together into the quartz fluorometer cells and kept at 37°C for 5 minutes. After the ABAP is added, fluorescence is measured at 495 nm and monitored every 5 minutes. The modified TRAP method produces a linear decrease in fluorescence due to the thermal decomposition of ABAP like the original oxygen probe-based method. A period of total protection is indicated by a lag phase upon the addition of any antioxidant compound, however, it is assumed that the total plasma antioxidant capacity is directly related to the length of the lag phase. The TRAP is quantified by comparing the lag phase produced by the antioxidant compound to the lag phase produced by a Trolox® solution of known concentration.

The modified TRAP method does not measure the ability of plasma to break the lipid peroxidation chain triggered by ABAP, but it is not fully defined whether and to what extent lipid-soluble antioxidants are involved in TRAP. This modified TRAP method can only handle four to eight plasma samples at a time.

FRAP Assay. The Ferric Reducing Ability of Plasma (FRAP) assay was developed by Benzie and Strain and published in 1996, and is carried out on a COBAS FARA II spectorphotometric analyzer. FRAP reagent along with all solutions are freshly prepared each day. The FRAP reagent is heated to 37° C. A blank reading is taken, and then an antioxidant sample and water are added. Starting 0.5 seconds after the reaction is initiated, readings are taken every 15 seconds for the duration of the experiment. To determine the difference in absorbance, the change in absorbance measurement from the blank to that of the final measurement is calculated and then related to the change in absorbance of a standard iron(II) solution that is tested in parallel. The FRAP assay is not concentration-dependent, showing no deviation from the expected linear trend of the results going through the origin.

The FRAP assay has certain known problems, namely, there are no free radicals introduced into the system. The FRAP assay uses an oxidation/reduction reaction to measure the ability of a sample to reduce iron(III) to iron(II). An antioxidant donates electrons in the same manner as a reductant in an oxidation/reductions, so it is assumed that the FRAP assay is a method for evaluating antioxidant capacity. However, the FRAP assay does not directly measure the antioxidant capacity of a potential antioxidant. Also, since there are no free radicals introduced into the system, there is no way of comparing the antioxidant capacity towards different kinds of radicals. The FRAP assay cannot measure the antioxidant capacity of certain antioxidants accurately such as ascorbic acid, which can react with iron(II) and SH group-containing antioxidants. The FRAP assay does not take into account the quantity of inhibition; thus, FRAP leaves out an important component of the total antioxidant capacity. The inability of the FRAP and TEAC assays to accurately determine the antioxidant capacity is evident upon comparison of results from the two assays where there is no linear correlation.

Fluorescence-Based Methods. The Fluorescence-Based method is based on the discovery that the fluorescence of $\beta$-phycoerythrin changes with respect to time upon damage caused by peroxyl and hydroxyl radical attack. The $\beta$-phycoerythrin method uses a Perkin-Elmer MPF 44B fluorescence spectrophotometer to detect the fluorescence. A fluorescence measurement is given with respect to time and is used to determine the amount of protection that an antioxidant provides by observing how long there is a "flat period" with respect to a control. In these indirect, fluorescence-based methods if the level of absorbance stays the same, the "flat period" for a longer period of time for chemical A than it does for chemical B, then chemical A is said to protect from radical attack better than compound B and is therefore a stronger antioxidant.

The fluorescence method provides a method for quantitating the level of non-serum antioxidants in plasma or other biological fluids rapidly on small samples and has been used to analyze the antioxidant potential of plasma, proteins, DNA, neurotransmitters and related substances, vitamins and their derivatives, and other chemicals. However, the indirect fluorescence method contains some problems. For example, these assays measure the inhibition percent calculated from the initial linear rate of fluorescence loss instead of accounting for the inhibition time of, e.g., $\beta$-PE. The fluorescence method offers neither a way to determine the contribution of lipid-soluble antioxidants, nor to determine the contribution of protein in serum to the total antioxidant capacity.

Phosphomolybdenum Complex Assay (PCA). The formation of a phosphomolybdenum complex is similar to the FRAP method. The PCA method is based on the change in the absorbance after the reduction of molybdenum(VI) to molybdenum(V). To reduce the molybdenum(VI) to molybdenum(V), a reducing species must be present like, e.g., an antioxidant. Samples are prepared just before use by dissolution in the appropriate solvent. For water-soluble compounds, water is used. For substances soluble in organic solvents, ethanol, methanol, dimethyl sulfoxide, or hexane is used with exact concentrations determined from literature-based absorption coefficients. After being ground and frozen, seed samples are dissolved, and an extraction is performed if necessary. A sample is then mixed with the molybdenum-containing reagent solution. After a period of incubation and cooling, an absorbance measurement is taken against a blank using, e.g., a UV-visible spectrophotometer. In unknown samples, organic and water-soluble antioxidant capacities are expressed as equivalents of $\alpha$-tocopherol and ascorbic acid, respectively. The antioxidant capacity is quantified on the basis of comparison of the molar absorption coefficient of the phosphomolybdenum complex. The closer the molar absorption coefficient is to one, the better the antioxidant. The phosphomolybdenum method is a good, simple method for determining the antioxidant potential of stronger antioxidants such as vitamin E from 25-37°C. This method is an inexpensive alternative to other available methods for determining the total antioxidant potential.

Traditional ORAC Assays. The Oxygen-Radical Absorbance Capacity (ORAC) methods use the chemical properties of phycoerythrins, fluorescing proteins. The ORAC assay differs from the Glazer method in that the reaction is driven to completion in the ORAC method while, as stated earlier, the Glazer method looks at what is reported as the flat period. The ORAC method can use serum with proteins removed by treatment with ammonium sulfate, followed by ultracentrifugation. The peroxyl radical generator, 2,2'-azobis(2-amidinopropane)dihydrochloride (AAPH), is used in this assay.

Traditional ORAC method detection is performed using, e.g., a Perkin-Elmer LS-5 fluorescence spectrophotometer until zero fluorescence occurs. The reagents are added to the cuvettes, and following the addition of AAPH, the reaction mixture is incubated at 37°C, and fluorescence measurements are taken every 5 minutes through completion of the reaction. The results are reported as the ORAC value, which refers to the net protection area under the quenching curve of $\beta$-PE in the presence of an antioxidant. The ORAC value is calculated by dividing the area under the sample curve by the area under the Trolox® curve with both areas being corrected by subtracting the area under the blank curve. One ORAC unit is assigned as being the net protection area provided by 1 $\mu$M Trolox® in final concentration. When the area under the curve for the sample is compared to the area under the curve for Trolox®, the result is given in Trolox® equivalents.

An automated ORAC method uses, e.g., a COBAS FARA II centrifugal analyzer. After the initiator is added, fluorescence measurements were taken after 0.5 seconds and then every 2 minutes following the initial reading. The COBAS FARA II is equipped with a centrifuge which allows for spinning and mixing of samples. The COBAS FARA II is able to handle up to 30 samples at one time and the results are reported using the area under the curve to determine the ORAC value as they were in the original method. The COBAS FARA II method has been used effectively to evaluate several sample matrices.

The ORAC may be further modified by using a fluorescein salt instead of $\beta$-phycoerythrin. $\beta$-phycoerythrin is approximately 30% pure due to the isolation process and is inconsistent from lot to lot. It was determined that due to variable reactivity to peroxyl radicals, $\beta$-phycoerythrin also produces an inconsistency from lot to lot. Also, $\beta$-phycoerythrin can be photobleached after exposure to excitation light for a certain time. Due to nonspecific protein binding, $\beta$-phycoerythrin interacts with polyphenols which affects stability. Also, $\beta$-phycoerythrin is also much more expensive than fluorescein. For these reasons fluorescein was used in place of $\beta$-phycoerythrin.

The ORAC method was adapted to be able to analyze the lipid-soluble antioxidant samples by introducing randomly methylated beta-cyclodextrin (RMCD) in 50% acetone-water mixture, see e.g. Huang D et al., J. Agric. Food Chem. 2002, 50, 1815-1821. This acetone: water mixture made lipid-soluble antioxidants soluble in phosphate buffer. The ORAC method is a simple, sensitive, and reliable way to measure the peroxyl radical absorbing capacity of antioxidants and serum or other biological fluids. Hydroxyl radical absorbing capacity of serum has been performed successfully using the ORAC method. The ORAC(fl) method can be used with a fluorometry microplate reader using a 96-well plate to perform simultaneous kinetic analysis of many samples and to reduce the amount of serum sample required. The ORAC method is unique in its analysis in that it takes into account the inhibition time and degree of inhibition into a single quantity by measuring the area under the curve. The ORAC method is not affected by dilution. A comparison was run with the COBAS FARA II automated assay using β-phycoerythrin with the FRAP and TEAC assays, and there was no linear correlation between the ORAC and the TEAC, further indicating the inability of the TEAC assay to accurately determine the antioxidant capacity of a sample. There was, however, a rather weak linear correlation between the ORAC and the FRAP, which shows the low precision of the FRAP assay to determine the antioxidant capacity of a potential antioxidant.

Further assays for testing the antioxidant activity of a natural product (aqueous spinach extract) are described in Bergman M et al., Phytochemistry 58 (2001) 143-152.

Several methods for determining the antioxidant potential of substances have been reviewed. All these methods have benefits, but they also have limitations. The high cost of these methods (including equipment-some of which is no longer manufactured-reagents, personnel costs etc.) makes them impractical for use by the majority of the small companies that make up the antioxidant industry.

Other methods include, e.g., U.S. Patent No. 5,518,590, issued to Fang, which discloses electrochemical sensors for motor oils and other lubricants. Briefly, a sensitive and rapid electrochemical sensor monitors motor oil deterioration, particularly antioxidation property, by determining the antioxidant and antiwear agent level remaining in an oil formulation. The electrochemical sensor is a two- or three-electrode electrochemical cell having a conductive electrolyte liquid or gel-like interphase over the electrode surfaces. The degree of deterioration of motor oil is monitored by measurements of antioxidation or antiwear capacity of the oil. The electrochemical sensor is used for monitoring other lubricants and hydrocarbons which contain electroactive additives. The electrochemical sensor allows measurements to be performed in-situ, without any chemical or physical pretreatment of the oil.

Another example of an antioxidant sensor is shown in U.S. Patent No. 6,638,415, issued to Hodges, which discloses a device and method for measuring the level of an oxidant or antioxidant analyte in a fluid sample. The device includes a disposable electrochemical cell, such as a thin layer electrochemical cell, containing a reagent capable of undergoing a redox reaction with the analyte. When the device and method are used with slow-reacting analytes, heat may be applied to the sample by a resistive heating element in the device or by an exothermic material contained within the electrochemical cell. The application of heat is taught to accelerate the rate of the redox reaction between the reagent and the analyte, which facilitates the electrochemical measurement of slow-reacting analytes.

Finally, U.S. Patent Application No. 20020182736, discloses methods to indirectly measure lipophilic antioxidant activity. A selective method for measuring lipid antioxidant activity within a lipid compartment of a sample using lipophilic radical generators and oxidizable lipophilic indicators is disclosed. The invention is said to accurately and efficiently determine the total antioxidant activity of a sample in both lipid and aqueous compartments. The methods of the invention can be used for diagnosing and protecting against disorders that arise from excess free radicals present in a subject. The reagents used in the methods of the invention can also be provided in a kit assay. However, the Oxygen Radical Absorbance Capacity (ORAC) value is measured indirectly using standard fluorescent probes.

## SUMMARY OF THE INVENTION

The present invention includes antioxidant sensors and methods that measure directly total sample oxidation levels and the effect of anti-oxidants on total sample oxidation state. Disclosed are also compositions and methods that are useful for providing effective amounts of anti-oxidants to an individual for optimal health. The apparatus and method disclosed herein detect the total anti-oxidant capacity of a sample, concurrently and directly in real-time. The present inventors recognized that the art has artificially created two mutually exclusive categories of anti-oxidants (lipophilic and lipophobic) and has measured them separately. Furthermore, the art has also generally measured the existence of the radicals indirectly, that is, using a detectable reporter molecule.

The present invention overcomes the limitations in prior art detectors and methods by using a rapid, inexpensive and direct detection system. To address these limitations, the present inventors developed the Oxygen Radical Absorbance Capacity-Oxygen (ORAC(o)) apparatus and method disclosed herein. Using the ORAC(o) apparatus, the present inventors were able to measure, for the first time, the effect of both lipophilic and lipophobic anti-oxidants on dissolved oxygen levels, concurrently and in real-time. Using the ORAC(o) assay, the inventors were also able to develop a synergistic anti-oxidant composition, which may be used alone or in combination with one or more anti-oxidant potentiators.

More particularly, the present invention includes an apparatus for detecting directly the antioxidant activity of both lipophilic and lipophobic anti-oxidants that includes a dissolved oxygen sensor in fluid communication with a sample and an oxygen radical sensitive molecule in a solvent/water/surfactant mixture; wherein the oxygen radical sensitive sensor concurrently detects both lipophilic and lipophobic anti-oxidants in the solvent/water/surfactant mixture. The oxygen radical sensitive molecule may be molecules that will react with oxygen, e.g., molecules with conjugated double bonds; or Nitrogen or Sulfur containing compounds. Examples of oxygen radical sensitive molecule, e.g., fluorescein, β-Phycoerythrin (β-PE), glutathione-S-transferase, linoleic acid or combinations thereof. The oxygen radical level is determined directly using a dissolved oxygen meter or sensor in a solvent/water/surfactant mixture. The level of dissolved oxygen is determined using an oxygen sensor, e.g., an electrochemical, a chemiluminescent, a surface plasmon resonance, an infrared, a capacitance coupled, a dye-coupled fiber optic or a hyperspectral oxygen sensor. The dissolved oxygen meter or sensor may be placed in-line for high-throughput analysis, may be a single sample detector and/or be adapted for office or even home use. The solvent is an organic solvent, acetone. The surfactant is a non-ionic detergent such as Tween-20. The solvent/water/surfactant volume ratio is 1:1:1.

The apparatus may further include one or more processors, e.g., a computer that may: control the detector, capture data, store data, perform calculations based on the data and/or a database of information, and/or display the data or summaries of the data in the form of tables, graphs, charts and the like. The processor/computer may also be connected and even control a fluidic system that is in fluid communication with the oxygen sensor and the solvent/water/detergent mixture. The present invention measures an area under the curve that relates the relative disappearance of oxygen that results from the activity of the sample being tested for anti-oxidant capacity to the relative disappearance of oxygen observed as a result of the activity of a known standard. Using the present invention, and the methods described herein, the level of dissolved oxygen is measured directly in the solution that include both lipophilic and lipophobic anti-oxidants, concurrently. An examples of the formula for calculating the AUC may be:

$$ORAC(o) = \frac{AUC_{SMP}-AUC_{BLNK}}{AUC_{TRLX}-AUC_{BLNK}} \cdot \frac{X\ 1000\ (mg/gr)\ X\ [TRLX\ (\mu mol/ml)]}{[SMP\ (mg/ml)]}$$

wherein $AUC_{SMP}$ is the area under the curve value of the sample;
wherein $AUC_{BLNK}$ is the area under the curve value of the blank;
wherein $AUC_{TRLX}$ is the area under the curve value for Trolox®; and
wherein SMP is the sample.

The present invention also includes a method of determining directly the anti-oxidant activity including the steps of: determining the dissolved oxygen level in a test solution dissolved in said solvent/water/surfactant mixture in the presence of one or more anti-oxidants and an oxygen radical sensitive molecule, wherein both lipophilic and lipophobic anti-oxidant activity is measured with an oxygen detector. The dissolved oxygen level is determined using an oxygen detector, e.g., electrochemical, chemiluminescent, surface plasmon resonance, capacitance coupled, a dye-coupled fiber optic or a hyperspectral oxygen sensor. The anti-oxidant activity may be measured at about 37 degrees Centigrade. Examples of radical intiators include, e.g., 2,2'-azobis[2-(5-methyl-2-imidazolin-2-yl)propane]dihydrochloride, 2,2' azobis (2-amidinopropane)dihydrochloride (AAPH), 2,2'-azobis(2-amidinopropane)[2-(N-stearyl)amidinopropane] dihydrochloride (SA-1), 2,2'-azo(2-(2-imidiazolin-2-yl)-propane)-[2-[2-[4-n-octyl]imidazolin-2-yl]-propane] dihydrochloride (C-8), 2,2'-azobis(4-methoxy-2,4-dimethylvaleronitrile) (MeO-AMVN), 2,2'-azobis(2,4-dimethylvaleronitrile) (AMVN), azo-bis-isobutylnitrile, 2,2'-azobis (2-methylproprionate) (DAMP), and 2,2'-azobis-(2-amidinopropane), salts or mixtures thereof. The detector may even be disposable.

The present disclosure also includes a dietary supplement that includes any isolated and purified lipophobic anti-oxidant and any isolated and purified lipophilic anti-oxidant, wherein the lipophobic and the lipophilic antioxidants combined have a dissolved oxygen value of greater than 6,000 µMol Trolox® Equivalents (TE)/gram. This dietary supplement does, however, not form part of the instant invention. The skilled will recognize that the value may also be expressed as liquid equivalents, e.g., 6,000 µMol Trolox® Equivalents (TE)/milliliter. The lipophobic and the lipophilic anti-oxidant are time-released and may include one or more vitamin E selected from alpha, beta, delta, epsilon, gamma, zeta, eta, xi1, xi2, and sigma tocopherols, and alpha, beta, delta and gamma tocotrienols, analogs thereof, pharmaceutically acceptable salts thereof, and combinations thereof. Examples of lipophilic anti-oxidants include quercetin, kaempferol, myricetin, apigenin and derivates, analogs, pharmaceutically acceptable salts thereof, and combinations thereof.

The dietary supplement that includes any isolated and purified lipophobic anti-oxidant and any isolated and purified lipophilic anti-oxidant, may also include two or more essential saccharide, e.g., galactose, galactosamine, glucosamine, glucose, mannose, acetylated-mannose, N-acetylneuraminic acid, fucose, N-acetylgalactosamine, N-acetylglucosamine and/or xylose. In one embodiment, the supplement also includes source of vitamin C, e.g., a plant source with a high level of natural, bioavailable vitamin C such as an Australian bush plum (*Terminalia ferdinandiana*). In another specific embodiment, the Vitamin C is a potentiator of anti-oxidant activity from a plant source of vitamin C such as wild-Australian bush plum (*Terminalia ferdinandiana*), which has a higher percentage of Vitamin C than farm grown bush plum. The supplement may also include one or more pro-biotics, e.g., *Lactobacillus sp.* and *Bifidobacterium sp.* The supplement may be compressed to provide a generally oxygen impermeable surface, e.g., a roller-compressed particle, a capsule, a tablet, a mini-tab, a caplet, an effervescent tablet or combinations thereof.

As a point of comparison with the ORAC(o) apparatus and method described hereinabove, the isolated and purified lipophilic and lipophobic anti-oxidants, will have an anti-oxidant ORAC(fl-lipo) value greater than 7000 μMol Trolox® Equivalents (TE)/gram. The present invention was used in an open label study to measure the change in anti-oxidant levels in each individual taking an anti-oxidant/glyconutrient blend that included the anti-oxidants of the present disclosure in their diets. When provided to a patient, the lipophobic and the lipophilic antioxidants provided an average increase of over 13% as measured by ORAC(β-PE) from the cumulative patient population's average baseline antioxidant level.

The present disclosure also includes a number of compositions. The compositions disclosed herein are based on the recognition that dietary supplements available currently fail to combine lipophilic and lipophobic anti-oxidants with measurable activities above those expected from the individual components. Using the apparatus and method of the present invention, the present inventors were able to develop synergistic combinations of not only lipophilic and lipophobic anti-oxidants, but also add potentiators of the anti-oxidant activity.

Flavonoids like quercetin have recently been shown to increase the transcription of the heavy subunit of the rate-limiting enzyme in glutathione synthesis, gamma-glutamylcysteine synthetase, through the gene's antioxidant responsive elements. The increased transcription is subsequently translated in increased intracellular levels of reduced (active) glutathione in tissue culture cells. Quercetin is a major component of red wine, grape skin, and onions. Studies of quercetin from red wine, grape skin, and onions suggest beneficial effects upon health. Quercetin has been shown to be well absorbed by humans. One study showed that all ingested quercetin was metabolized two hours following a meal (European Research on Functional Effects of Dietary Antioxidants, September 25-28, 2002, Cambridge, UK). Upon finding substantially decreased LDL oxidation in subjects consuming moderate amounts of red wine, researchers asserted that the protective effects could very likely be attributed to the activity of quercetin and its metabolites. Quercetin has also been shown to enhance the stability of erythrocytes.

The present inventors recognized that due to the numerous and diverse factors influencing oxidative stress, anti-oxidant supplementation would need to be tailored to an individual's need and chemistry. Furthermore, it was recognized that a combination of tocopherols was required to meet the differing needs of individuals. The combination of tocopherols is necessary because some antioxidants are "selected" by the body. For example, the predominant form of vitamin E in the North American diet is gamma-tocopherol, which is commonly found in vegetable oils as well as products derived form soybeans and corn. However, the body predominantly retains alpha-tocopherol. A specific method, leaning on alpha-tocopherol transfer protein has been found to regulate the concentration of alpha-tocopherol in the body. Unlike anti-oxidant compositions in the prior art, the present disclosure uses mixed tocopherols to provide the body with many forms of vitamin E, allowing for the selection, retention and use of the optimal amounts of each form. Furthermore, the synergistic combination of quercetin and mixed tocopherols provides the body with a wide array of antioxidant nutrients optimal selection, which may differ based upon an individual's needs.

The present inventors sought to maximize further the activity of the synergistic quercetin and mixed tocopherol combination by adding compounds that help potentiate the activity of these anti-oxidants. One such potentiator is Vitamin C. Vitamin C has significant antioxidant activity attributed to it as well as several non-antioxidant nutritive functions. Many attribute pro-oxidant properties to vitamin C, especially in the presence of transition metals. The pro-oxidant properties of vitamin C may not be entirely destructive. However, other researchers assert that they found that vitamin C behaves as an antioxidant, even in the presence of unbound metals.

Other potentiators of anti-oxidant activity include natural extracts, such as grape seed extract and green tea extract. In one study, green tea exhibited potent antimutagenic activity in vitro and inhibited the development of carcinogen-induced preneoplastic lesions in the rat colon. Green tea also significantly inhibited the formation of intestinal polyps. Therefore, the present inventors combined not only a synergistic combination of purified and isolated anti-oxidants from natural sources, such as quercetin and mixed tocopherols, but further added potentiators that increased the detectable anti-oxidant activity of these agents.

More particularly, the compositions a dietary supplement that includes a nutritionally effective amount of two or more essential saccharides; an isolated and purified lipophobic oxygen-radical quencher; and an isolated and purified lipophilic oxygen-radical quencher, wherein the lipophobic and the lipophilic oxygen-radical quenchers combined have an oxygen-radical quencher value of greater than 6,000 μMol Trolox® Equivalents (TE)/gram. In one assay, the lipophobic and the lipophilic oxygen-radical quencher when provided to a patient provide an average increase of over 13% as measured by ORAC(fllipo) from the patient population's baseline antioxidant level. The lipophobic and the lipophilic oxygen-radical quencher are packaged for extended-release, and may include one or more of the following vitamin E molecules: alpha, beta, delta, epsilon, gamma, zeta, eta, xi1, xi2, and sigma tocopherols, and alpha, beta, delta and gamma tocotrienols, analogs thereof, pharmaceutically acceptable salts thereof, and combinations thereof. The lipophilic oxygen-radical quencher may include one or more of the following: flavonols, quercetin, kaempferol, myricetin, apigenin and derivates, analogs, pharmaceutically acceptable salts thereof, and combinations thereof. The supplement may further include two or more saccharides selected from the group consisting of galactose, glucose, mannose, N-acetylneuraminic acid, fucose, N-acetylgalactosamine, N-acetylglucosamine and xylose, derivates, analogs, pharmaceutically acceptable salts thereof, and combinations thereof.

BRIEF DESCRIPTION OF THE DRAWINGS

For a more complete understanding of the features and advantages of the present invention, reference is now made to the detailed description of the invention along with the accompanying figures and in which:

FIG. 1 depictions an ORAC(o) direct anti-oxidant apparatus;

FIG. 2 is a flowchart of the QRAC(o) method of the present invention;

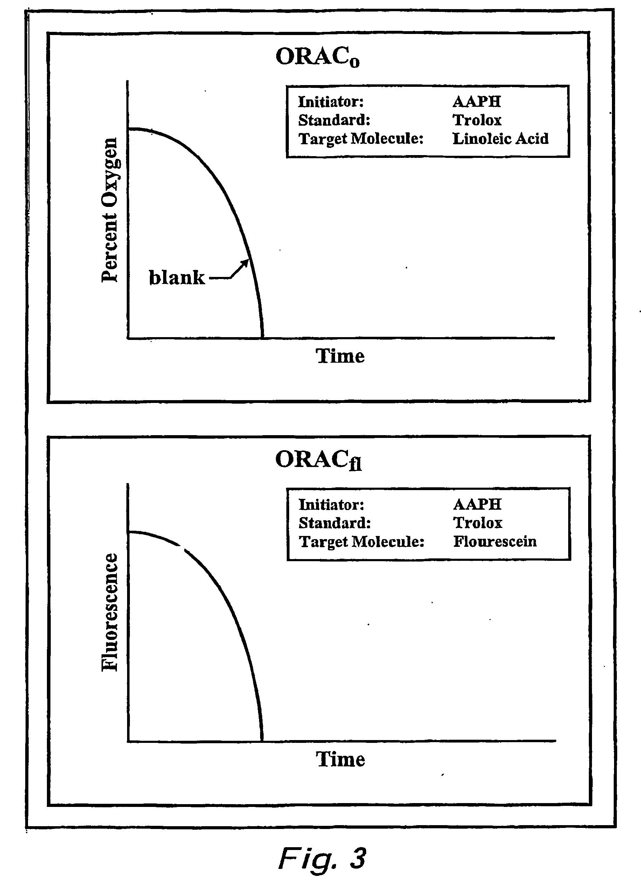

FIG. 3 are two graphs that compare the blank solution in an ORAC(o) (percent oxygen) with the ORAC(fl) (fluorescence) measurements using AAPH an initiator, Trolox® as the standard and linoleic acid or fluorescein, respectively as the oxygen radical targets;

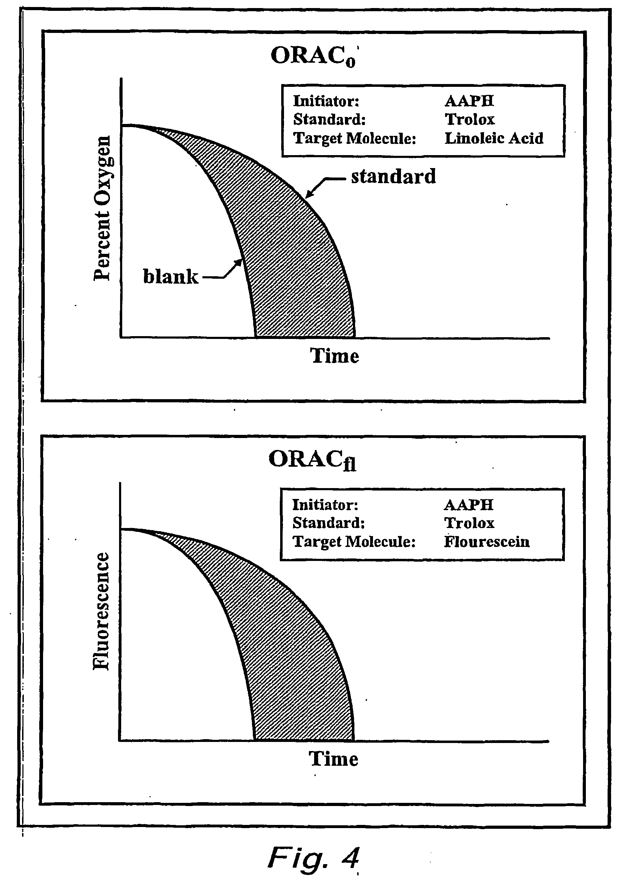

FIG. 4 are two graphs that compare the anti-oxidant standard Trolox® in an ORAC(o) (percent oxygen) with the ORAC(fl) (fluorescence) measurements using AAPH  an initiator, Trolox® as the standard and linoleic acid or fluorescein, respectively as the oxygen radical targets;

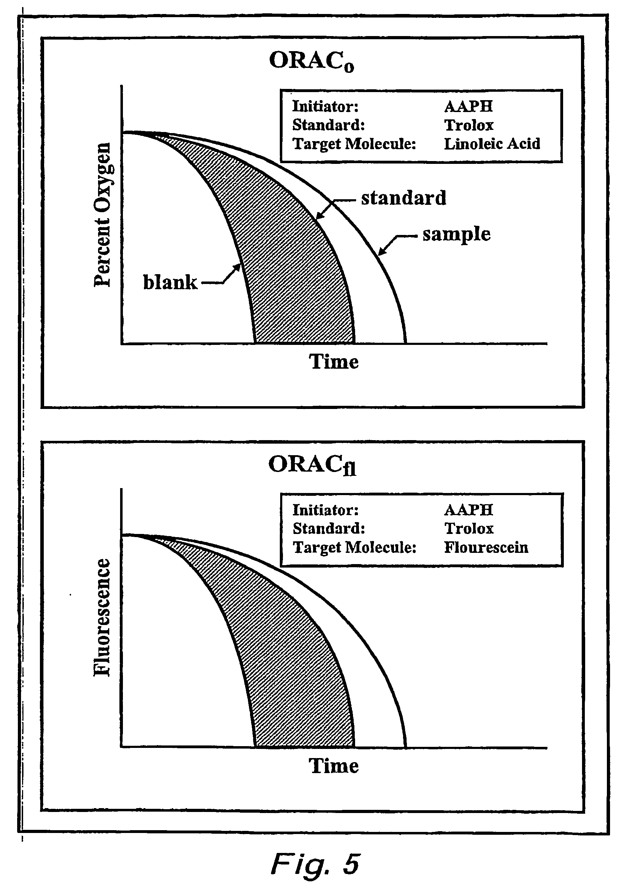

FIG. 5 are two graphs that compare the blank solution, the standard and the sample, in an ORAC(o) (percent oxygen) with the ORAC(fl) (fluorescence) measurements using AAPH as an initiator, Trolox® as the standard and linoleic acid or fluorescein, respectively as the oxygen radical targets;

FIG. 6 is a graph that shows an antioxidant effect of the combination of quercetin (Q) at 5 $\mu$g/mL and mixed tocopherols (MT) at 5 $\mu$g/mL as compared to each ingredient separately at a concentration of 10 $\mu$g/mL as measured by the ORAC(o) method of the present invention;

FIG. 7 is a graph that shows an antioxidant effect of the combination of quercetin (Q) at 5 $\mu$g/mL and mixed tocopherols (MT) at 5 $\mu$g/mL, and Trolox® as a control measured by the ORAC(o) method of the present invention;

FIG. 8 is a graph of the Area Under the Curve (AUC) results from titrated ratio of quercetin and mixed tocopherols using the ORAC(o) method to measure antioxidant capacity;

FIG. 9 is another graph of AUC results from titrated ratio of quercetin and mixed tocopherols using the ORAC(o) method to measure antioxidant capacity that demonstrated the expected results (line) and the extent of synergy detected above the line as compared to the expected results from titration of the quercetin and mixed tocopherols;

FIG. 10 is a graph that shows the results from ORAC(o) assays for varying ratios of grape skin extract and green tea extract in the presence of 49.18% quercetin, 32.79% mixed tocopherols, and 1.64% bush plum. The optimal ratio of grape skin extract to green tea extract is 60/40 to 80/20;

FIG. 11 is a graph that shows ORAC(fl) results of the combination of quercetin (Q) at 5 $\mu$g/mL and mixed tocopherols (MT) at 5 $\mu$g/mL as compared to each ingredient separately at a concentration of 10 $\mu$g/mL.

FIG. 12 is a graph of an ORAC(fl) assay measuring a titration of quercetin versus $\alpha$-tocopherol dissolved in acetone: water;

FIG. 13 is a graph of an ORAC(fl) assay measuring the anti-oxidant activity measured for a fixed ratio of quercetin: $\alpha$-tocopherol ratio dissolved in a solvent:water:detergent mixture;

FIG. 14 is a graph of an ORAC(fl) assay measuring the anti-oxidant activity measured for a fixed ratio of quercetin: $\alpha$-tocopherol ratio dissolved in two different rations of solvent:water to detergent mixtures;

FIG. 15 is a graph showing the ORAC(o) values obtained with different ratios of quercetin and mixed tocopherols;

FIG. 16 is a graph showing the ORAC(o) values for different ration of grape seed extract and green tea extract; and

FIG. 17 is a graph that shows the ORAC(o) values of the combination of the maximum quercetin:mixed tocopherol and the grape seed extract and green tea extract ratios.

DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention which is defined by the appended claims.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The present invention is based, in part, on the recognition that the art has artificially created two mutually exclusive categories of anti-oxidants and has measured them separately. Furthermore, the art has also generally measured the existence of the radicals indirectly, that is, using a reporter molecule. The present invention provides an apparatus and method that not only detects the total anti-oxidant capacity of a sample, concurrently, but does so directly.

As used herein, the "antioxidant" refers to any molecule that delays or prevents the oxidation of an oxidizable target molecule. Antioxidants act by: scavenging biologically important reactive free radicals or other reactive oxygen species (e.g., $O_2^-$, $H_2O_2$, HOCl, ferryl, peroxyl, peroxynitrite, and alkoxyl); preventing oxygen radical formation; or catalytically converting the free radical or other reactive oxygen species to a less reactive species. Antioxidants are generally divided into two classes: (1) lipid (lipophilic or hydrophobic) antioxidants; and (2) aqueous (lipophobic or hydrophilic) antioxidants. Examples of lipid antioxidants include, but are not limited to, carotenoids (e.g., lutein, zeaxanthin, $\beta$-cryptoxanthin, lycopene, $\alpha$-carotene, and $\beta$-carotene), which are located in the core lipid compartment, and tocopherols (e.g., vitamin E, $\alpha$-tocopherol, $\gamma$-tocopherol, and $\delta$-tocopherol), which are located in the interface of the lipid compartment, and retinoids (e.g., vitamin A, retinol, and retinyl palmitate) and fat-soluble polyphenols, e.g., quercetin. Examples of aqueous antioxidants include, but are not limited to, ascorbic acid and its oxidized form, "dehydroascorbic acid," uric acid and its oxidized form "allantoin," bilirubin, albumin and vitamin C and water-soluble polyphenols such as catechins, which have high affinity to the phospholipid membranes, isoflavones and procyanidins.

A commonly used method for detecting the relative levels of oxygen radicals and anti-oxidant capacity is the Oxygen Radical Absorbance Capacity (ORAC) assay. In known ORAC-type assays, the anti-oxidant value is measured indirectly by measuring the effect of an oxygen radical on, e.g., a fluorescent or other detectable molecule, that may of may not be a good target for oxidation by the particular oxygen radical. Generally, when antioxidants are added to a test sample, a detectable decrease in the amount of a free radical, such as superoxide, or a non-radical reactive oxygen species, such as hydrogen peroxide, may be seen in the sample, compared with a sample untreated with the antioxidant (i.e., control sample). However, these indirect methods monitor the change in antioxidant status via an intermediary (e.g., fluorescein, $\beta$-phytoerythrin ($\beta$-PE), etc.) measured with the assumption that the effect of the radical on the intermediary is a true reflection of the relative level of oxidants and anti-oxidants. Controls for these assays are known concentrations of oxygen radical generators and known antioxidants that are measured and used as standards for the samples.

As used herein, the term "free radical" refers to molecules containing at least one unpaired electron. Most molecules contain even numbers of electrons, and their covalent bonds normally include shared electron pairs. Cleavage of such bonds produces two separate free radicals, each with an unpaired electron (in addition to any paired electrons). Free radicals may be electrically charged or neutral, are highly reactive and usually short-lived. Free radicals combine with one another or with atoms that have unpaired electrons. In reactions with intact molecules, free radicals try to complete their own electronic structure, generating new radicals, which go on to react with other molecules creating a chain reaction. Free radical chain reactions are particularly important in decomposition of substances at high temperatures and in polymerization. In the body, oxidized free radicals are responsible for damage tissues. Heat, ultraviolet light, and ionizing radiation all generate free radicals. Free radicals are generated as a secondary effect of oxidative metabolism. An excess of free radicals can overwhelm the natural protective enzymes such as superoxide dismutase, catalase, and peroxidase. Free radicals such as hydrogen peroxide ($H_2O_2$), hydroxyl radical (HO·), singlet oxygen ($^1O_2$), superoxide anion radical ($O·_2^-$), nitric oxide radical (NO·), peroxyl radical (ROO·), peroxynitrite (ONOO$^-$) can be in either the lipid or aqueous compartments. Antioxidant nutrients (e.g., vitamins C and E, selenium, polyphenols) may reduce these effects.

As used herein, the phrase "lipid compartment" refers to compounds that have cyclic or acyclic long-chain aliphatic hydrocarbons and their derivatives, such as fatty acids, alcohols, amines, amino alcohols and aldehydes. For example, common lipids include fatty acids, fats, phospholipids, steroids, eicosanoids, waxes and fat-soluble vitamins. Some lipids may be generally classified into two groups, the simple lipids and the complex lipids, e.g., triglycerides or fats and oils, fatty acid esters of glycerol, waxes, fatty acid esters of long-chain alcohols and steroids such as cholesterol and ergosterol. Complex lipids include, e.g., phosphatides or phospholipids (phosphorous containing lipids), glycolipids (carbohydrate containing lipids), and sphingolipids (sphingosine containing lipids).

As used herein, the term "lipid" includes fats or fat-like substances. The term is descriptive rather than a chemical name such as protein or carbohydrate. Lipids include true fats (i.e., esters of fatty acids and glycerol), lipoids (i.e., phospholipids, cerebrosides, waxes) and sterols (i.e., cholesterol, ergostrol). Lipids can be a target of oxidation through mechanisms, such as autoxidation. As used herein, the term "fatty acid" refers to a group of, e.g., negatively charged, generally linear hydrocarbon chains. The hydrocarbon chains of fatty acids vary in length and oxidation states. Generally, fatty acids have a negatively charged portion (e.g., at the carboxyl end), and a "tail" portion, which determines the water solubility and amphipathic characteristics of the fatty acid. For example, fatty acids are components of the phospholipids that include biological membranes, as fats, which are used to store energy inside cells, or for transporting fat in the bloodstream. As used herein, the term "phospholipid" refers to any of the class of esters of phosphoric acid that include at least one of the following side-groups: a fatty acid, an alcohol and a nitrogenous base.

As used herein, the term "fat" or "fats" refers to any of the glyceryl esters of fatty acids, e.g., the monoacylglycerol, diacylglycerol and triacylglycerol forms of fatty acids. Triglycerides refer to those molecules that are neutrally charged and entirely hydrophobic, i.e., reduced molecules. Monoacylglycerides and diacylglycerides are metabolic intermediates in phospholipid synthesis, while triglycerides form the fat molecules that are used to store chemical energy in a water free, compact state. As used herein, the term "fat-soluble vitamins" refers to, e.g., common fat-soluble vitamins include vitamin (A) (retinol), Vitamin D (e.g., vitamin D3 (cholecalciferol)), Vitamin E, Vitamin K and the like.

As used herein, the phrase "lipid antioxidant activity" or "lipid antioxidant capacity" are used interchangeably and refer to the measurement of antioxidant ability arising from the lipid compartment of a sample. As used herein, the phrase "aqueous antioxidant activity" or "aqueous antioxidant capacity" are used interchangeably and refer to the measurement of antioxidant ability arising from the aqueous compartment of a sample. As used herein, the phrase "total antioxidant activity" or "total antioxidant capacity" are used interchangeably and refer to the measurement of antioxidant ability arising from both the lipid and aqueous portions of a sample.

As used herein, the phrase "aqueous compartment" refers the portion of a fluid sample that does not interact with the lipid compartment. The aqueous compartment includes biologic fluid samples such as blood, plasma, serum, feces, cerebral spinal fluid, amniotic fluid, interstitial fluid, lymphatic fluid and synovial fluid. For example, the aqueous compartment of a fluid sample such as serum may include not only the liquid portion that remains after blood has been allowed to clot and is centrifuged to remove the blood cells and clotting elements, but also other compounds such as: proteins, e.g., albumin and globulins; antibodies; enzymes; small amounts of nutritive organic materials, such as amino acids and glucose; inorganic substances such as sodium, chloride, sulfates, phosphates, calcium, potassium, bicarbonate, magnesium, iodine, zinc, and iron; small amounts of waste products, such as urea, uric acid, xanthine, creatinine, creatine, bile pigments and ammonia; and trace amounts of gases such as oxygen and carbon dioxide. The fluid sample may also be a non-biological sample, for example, chemical formulations, synthetic compositions, or food products and cosmetic products.

As used herein, the term "sample" refers to a liquid or fluid biological sample, or a solid biological sample in which free radicals can be generated using a free radical generator, (e.g., a lipophilic free radical generator or an hydrophilic free radical generator) and can be detected using the (ORAC(o)) detector and method of the present invention. Biological samples include, e.g., blood, plasma, serum, cerebral spinal fluid, urine, amniotic fluid, interstital fluid, and synovial fluid. Solid biological samples include, e.g., a tissue, cells, tissue culture, fixed cells, cell supernatants, or even portions (or extracts) of tissue or cell matter. The term sample also includes non-biological samples such as a chemical solution, synthetic composition, and food. As used herein, the terms "relative ORAC(o)" and "ORAC(o)" refer to the same value, which is measured by equivalence to micro-moles of Trolox® per gram or milliliter. A negative value ORAC (o) reflects less radical quenching activity than obtained with a blank which indicates that a composition is a pro-oxidant, i.e., an agent that promotes oxidation, rather than acting as an antioxidant.

As used herein, the phrases "radical generator" or "radical initiator" are used interchangeably and refer to an agent, compound or molecule that produces free radicals. The radical generator is capable of producing free radicals at a measured level, for example, at a level at which antioxidants or oxidizable indicators can interact with the free radicals to produce a measurable or detectable output. Examples of radical generators include, e.g., azo radical generators, which are compounds that produce a flux of free radicals at a known constant rate. Examples of azo radical generators include, e.g., 2,2'-azobis(4-methoxy-2,4-dimethylvaleronitrile) (MeO-AMVN), 2,2'-azobis(2,4-dimethylvaleronitrile) (AMVN), azo-bis-isobutylnitrile, 2,2'-azobis (2-methylproprionate) (DAMP), and 2,2'-azobis-(2-amidinopropane), 2,2'-azobis[2-(5-methyl-2-imidazolin-2 yl)propane]dihydrochloride, iron, ascorbic acid and metal ions.

As used herein, the "subject" refers to any living organism. The term subject includes, e.g., fish, mammals, reptiles, birds, insects and the like. Specific examples include: humans, non-human primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be covered.

As used herein, the phrase "free radical associated disorder" refers to a pathological condition of from the production of or exposure to free radicals. As used herein, the term "free radical associated disorder" includes pathological states where damage from free radicals contributes to the pathology of the disease state, or wherein administration of a free radical inhibitor (e.g., desferrioxamine), scavenger (e.g., tocopherol, glutathione), or catalyst (e.g., SOD, catalase) are shown to produce a detectable benefit by decreasing symptoms, increasing survival, or providing other detectable clinical benefits in protecting or preventing the pathological state. Examples of free radical disorders include, but are not limited to, ischemic reperfusion injury, inflammatory diseases, systemic lupus erythematosis, myocardial infarction, stroke, traumatic hemorrhage, spinal cord trauma, Crohn's disease, autoimmune diseases (e.g., rheumatoid arthritis, diabetes), cataract formation, age-related macular degeneration, Alzheimer's disease, uveitis, emphysema, gastric ulcers, oxygen toxicity, neoplasia, undesired cell apoptosis and radiation sickness.

As used herein, the term "oxidative stress" refers to the level of damage produced by oxygen free radicals in a subject. The level of damage depends on how fast reactive oxygen species are created and then inactivated by antioxidants as well as the location and speed of repair. As used herein, the term "deviation" or "deviate" as related to oxidative state and oxidative stress are used interchangeably and refer to a change in the antioxidant activity of a sample. The change in oxidative state can be an increase, decrease, elevation or depression of antioxidant activity from a known normal value. For example, an increase or decrease of antioxidant activity in the lipid compartment of a sample, the aqueous compartment of a sample, or in both the lipid and aqueous compartment of the sample.

As used herein, the term "essential saccharides," is used to define the monosaccharides commonly found in the oligosaccharide chains of cellular glycoproteins and which may not be readily available through diet or biochemical manufacture in the human body (see, e.g., Harper's Biochemistry (Murray et al., 1996)(listing eight) and Principles of Biochemistry, Vol II (Zubay, et al., 1995)(listing eleven). While over 200 monosaccharides have been found in nature, these eleven are believed to be important toward maintaining good health in mammals: galactose, glucose, mannose, N-acetylneuraminic acid, fucose, N-acetylgalactosamine, N-acetylglucosamine, xylose, iduronic acid, arabinose and glucuronic acid. The structures of these carbohydrates are well-known (see, e.g., Stryer's Biochemistry (Stryer, 1995) and the Merck Index, 12th Edition, 1996).

As used herein the term "nutritionally effective amount" is used to define the amount that will provide a beneficial nutritional effect or response in a mammal. For example, as nutritional response to vitamin- and mineral-containing dietary supplements varies from mammal to mammal, it should be understood that nutritionally effective amounts of the vitamins and minerals will vary, respectively. Likewise, the lack of an essential amino acids, vitamin-C, iron, iodine, vitamins, minerals, carbohydrates, lipids and the like are known to affect physiological and cellular functions. A nutritionally effective amount of the anti-oxidants and saccharides disclosed herein serve to preserve and/or elevate the levels of these critical nutrients in the diet of, e.g., a human that seeks to maintain or augment their diet for these nutritional supplements. Thus, while one mammal may require a particular profile of vitamins and minerals present in defined amounts, another manunal may require the same particular profile of vitamins and minerals present in different defined amounts.

As used herein, the term "pharmaceutically acceptable salt" is used to describe those salts that are, within the scope of sound medical judgment, suitable for use in, on or with the tissues of humans and lower animals without undue toxicity, irritation, allergic response and the like and are commensurate with a reasonable benefit/risk ratio. Pharmaceutically acceptable salts are well-known in the art (see e.g., S. M. Berge, et al., J. Pharmaceutical Sciences, 1977). Suitable salts may be prepared during the final isolation and purification of the compounds disclosed or separately by reacting a free base function with a suitable organic acid. Representative acid addition salts include, but are not limited to acetate, adipate, alginate, citrate, aspartate, benzoate, benzene sulfonate, bisulfate, butyrate, camphorate, camphor sulfonate, digluconate, glycerophosphate, hemisulfate, heptanoate, hexanoate, fumarate, hydrochloride, hydrobromide, hydroiodide, 2-hydroxyethansulfonate (isothionate), lactate, maleate, methane sulfonate, nicotinate, 2-naphthalene sulfonate, oxalate, palmitoate, pectinate, persulfate, 3-phenylpropionate, picrate, pivalate, propionate, succinate, tartrate, thiocyanate, phosphate, glutamate, bicarbonate, p-toluene sulfonate and undecanoate. Examples of basic nitrogen-containing groups that are used as quatemizing agents include: lower alkyl halides (methyl, ethyl, propyl, and butyl chlorides, bromides and iodides); dialkyl sulfates (dimethyl, diethyl, dibutyl and diamyl sulfates); long chain halides (decyl, lauryl, myristyl and stearyl chlorides, bromides and iodides); arylalkyl halides (benzyl and phenethyl bromides) and the like. Examples of acids that may be employed to form pharmaceutically acceptable acid addition salts include inorganic acids, e.g., hydrochloric acid, hydrobromic acid, sulphuric acid and phosphoric acid and such organic acids as oxalic acid, maleic acid, succinic acid and citric acid. Basic addition salts can also be prepared in situ during the final isolation and purification of anti-oxidant compounds disclosed herein with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, and ethylammonium among others. Other representative organic amines useful for the formation of base addition salts include ethylenediamine, ethanolamine, diethanolamine, piperidine, piperazine and the like.

As used herein, the term "potentiate" refers to one or more agent that act directly or indirectly to increase or enhance the activity of the lipophobic and/or lipophilic anti-oxidants of the present disclosure. One such potentiator is Vitamin C, which may act to reactivate or recycle the anti-oxidants and may itself have significant antioxidant activity. The pro-oxidant properties of vitamin C have been observed in the presence of transition metals. Other potentiators of anti-oxidant activity include natural extracts, such as grape seed extract and green tea extract. In one study, green tea exhibited potent antimutagenic activity in vitro and inhibited the development of carcinogen-induced preneoplastic lesions in the animal model. As such, the potentiator enhace or may even synergize with the purified and isolated anti-oxidants from natural sources, such as quercetin and mixed tocopherols.

As used herein, the terms "glyconutritional" or "glyconutrient" refer to complex carbohydrates or saccharides or simple sugars that are synthesized in nature and are necessary for the biochemical synthesis of various classes of communication and signal molecules that may be free in interstitial cellular fluids, active in cell to cell communication (i.e., cytokines, growth factors, etc.), or constitute the molecular configuration comprising foci of highly specific molecular activity of cell membranes (i.e., receptor sites, ion-transport channels, antigenic identification, and the like).

As used herein, the terms "phytonutritional" or "phytonutrient" refer to naturally synthesized molecules found only in plants that are produced to protect the plant's cells. Phytonutrients primarily have antioxidant, free-radical scavenger and vital micronutrient activity. These molecules, supplied through dietary supplementation, are found in mature plant tissues, and are most concentrated in seed coats and fruiting tissues surrounding the seed. In mammalian tissues, these molecules, when supplied in the diet, are active in optimizing the biochemistry, immunology and physiology in the cellular microenvironment.

As used herein, the terms "plant extract" and "herbal extract" are used interchangeably to refer to phytochemicals that are produced in plant tissues and that can be extracted by water, polar, or petroleum solvents, and that have some degree of beneficial health or therapeutic activity. Most herbal agents can be toxic, especially when concentrated, but are generally safe when utilized in their more traditional manner in teas and poultices as a "folk medicinal for the treatment of disease and promotion of good health." As used herein, the term "herbal body-toning agent" refers to substances that have been observed by the inventors to reduce and reverse elastic tissue and collagen fiber damage caused by aging or sun-damage as evidenced by a restoration of skin turgor and elasticity which effectively reduces or eliminates wrinkles, sagging, hyperpigmentation and reversal of other undesirable elements of lost cosmetic appearance.

The carbohydrates included in the dietary supplement disclosed are available from a wide variety of natural and synthetic sources such as shrubs, trees, plants, yeasts, fungi, molds, gums, resins, starch and cellulose derivatives and natural mucin sources. Specifically, some of the natural sources include: (a) shrub or tree exudates which contain acacia, karaya, tragacanth, or ghatti; (b) marine gums which include agar, algin, or carrageenan; (c) seed gums which include guar, locust bean, or psyllium; (d) plant extracts which contain pectins or acetylated polymannose; (e) starch and cellulose derivatives such as hetastarch, carboxymethylcellulose, ethylcellulose, hydroxypropyl methylcellulose, methylcellulose, oxidized cellulose; and microbial gums which contain dextrans, xanthan. However, it should be recognized that the composition disclosed is not intended to be limited by the source from which the respective carbohydrates are obtained.

The saccharides disclosed can be found in nature as mono-, oligo- and/or polysaccharides. Thus, the compositions disclosed can contain the saccharides in their monomeric, oligomeric and/or polymeric forms. For a list of known natural sources for the saccharides and their uses, please refer to U.S. Patent Application No. US2003072770.

As used herein, the term "carbohydrate" is used interchangeably with the terms "saccharide," "polysaccharide," "oligosaccharide" and "sugar" the definitions of which are well known to those skilled in the art of carbohydrate chemistry. Althpugh the compositions disclosed are intended to include at least two or more essential saccharides, it should be noted that the saccharides can be in the form of mono-, oligo- and/or polysaccharides, e.g., a composition containing gum tragacanth and guar gum will be considered as containing galacturonic acid, sialic acid, mannose and galactose. Therefore, by controlling the amount of particular gums in a given dietary supplement, one can control the amount of the respective saccharides in the dietary supplement.

As used herein, the term "a mixture of at least two forms of vitamin E" is used to describe a mixture of at least two forms of tocopherol selected from alpha, beta, delta, epsilon, gamma, zeta, eta, xi1, xi2, and sigma tocopherols, and alpha, beta, delta and gamma tocotrienols, and combinations or derivatives thereof. In one embodiment, "a mixture of at least two forms of vitamin E" is a mixture of at least two forms of tocopherol selected from alpha, beta, delta, and gamma tocopherol. In another embodiment, "a mixture of at least two forms of vitamin E" is a mixture of alpha, beta, delta, and gamma tocopherol. "A mixture of at least two forms of vitamin E" may be obtained from VITAECAPS, SA, Spain, from Henkel Corporation; or from Cognis Corporation (Kankakee, IL), for example. COVITOL® F-350M is commercially available from Cognis and contains natural source alpha-tocopherol with mixed tocopherols which are obtained from edible vegetable oils. The particular mixture of tocopherols included in the antioxidant composition disclosed is determined by running an ORAC(o) antioxidant determination.

Salts or derivatives of tocopherols include pharmaceutically acceptable salts such as acetate, sulfate, succinate, nicotinate, allophanate, phosphate, quinone, or halogenated derivatives; esters; stereoisomers; and the like. The disclosure encompasses the use of vitamin E derivatives in which substitutions, additions, and other alterations have been made in the 6-chromanol ring and/or side chain, with the proviso that the derivatives maintain antioxidant activity of a vitamin E. For example, tocopherols and their derivatives can vary by the number and position of alkyl groups, double bonds and other substituents and variations on the ring and side chain. An "alkyl" is a cyclic, branched or straight chain chemical group containing only carbon and hydrogen, such as methyl, butyl, and octyl. Alkyl groups can be either unsubstituted or substituted with one or more substituents, e.g., halogen, alkoxy, acyloxy, amino, hydroxyl, mercapto, carboxy, or benzyl. Alkyl groups can be saturated or unsaturated at one or several positions. Typically alkyl groups will comprise 1 to 8 carbons, 1 to 6, or 1 to 4 carbon atoms. Additional tocopherols can be constructed by conjugation to the ring structure or side chain of various other moieties, such as those containing oxygen, nitrogen, sulfur and/or phosphorus. Tocopherol derivatives can also be made by modifying the length of the side chain from that found in prototypical tocopherols such as alpha-, beta-, delta- and gamma-tocopherol. Tocopherols can also vary in stereochemistry and saturation of bonds in the ring structure and side chain.

Additional tocopherol derivatives, including prodrugs, can be made by conjugation of sugars or other moieties to the side chain or ring structure. Mixed tocopherols include without limitation mixtures of stereoisomers of a single tocopherol (e.g., +and - stereoisomers of alpha-tocopherol; (+/-) indicates a racemic mixture) or mixtures of structurally distinct tocopherols (e.g., alpha- plus gamma-tocopherol).

Although the present disclosure includes the above cited eleven essential saccharides, it should be noted that other saccharides, nutritional compounds or biologically active or inert compounds may be included in the dietary supplement. Such other nutritional compounds include any one or more of phytonutrients, dioscorea complex, plant extracts, herbal extracts, plant parts, herbal components, vitamins or minerals. These nutritional compounds can be added to the dietary supplement, or they can be provided separately to a mammal being administered the dietary supplement. For example, a person receiving the glyconutrient-containing dosage form can also receive a phytonutrient in either the same or a separate dosage form. Inert compounds can include flavors, fillers, lubricants, buffers, gels, binders, excipients, carriers and/or other such compounds that facilitate the formulation or administration of the dietary supplement. All of the glyconutrient containing dietary supplement compositions disclosed, even those containing additional compounds, agents or other substances, can be obtained directly from Mannatech, Inc. (Coppell, Tex.).

The present invention includes an apparatus and method for directly and concurrently measuring the oxygen radical absorption capacity (ORAC) of a composition that includes both hydrophobic and/or hydrophilic antioxidants. The term "ORAC(o)" is used since the assay measures the ability of antioxidants to quench radicals by directly tracking the disappearance of oxygen in an oxygen radical absorption capacity assay the directly measures oxygen content in a sample (ORAC(o)). Current industry standard assays such as ORAC(fl) and ORAC(β-PE), measure antioxidant capacity by indirectly measuring degradation of fluorescent emissions of a fluorescent compound (fluorescein or β-phycoerythrin) upon exposure to oxygen radicals. These assays work well with hydrophilic antioxidants, but have limited effectiveness in measuring hydrophobic antioxidants or mixtures of hydrophobic and hydrophilic antioxidants. Furthermore, unlike the known ORAC(fl) and ORAC(β-PE) systems, the ORAC(o) disclosed herein is suitable for simplification and manufacturing as a disposable sensor. The system is robust enough to even permit the manufacture of an office and even a home system for immediate evaluation of oxidative capacity of a user from biological samples.

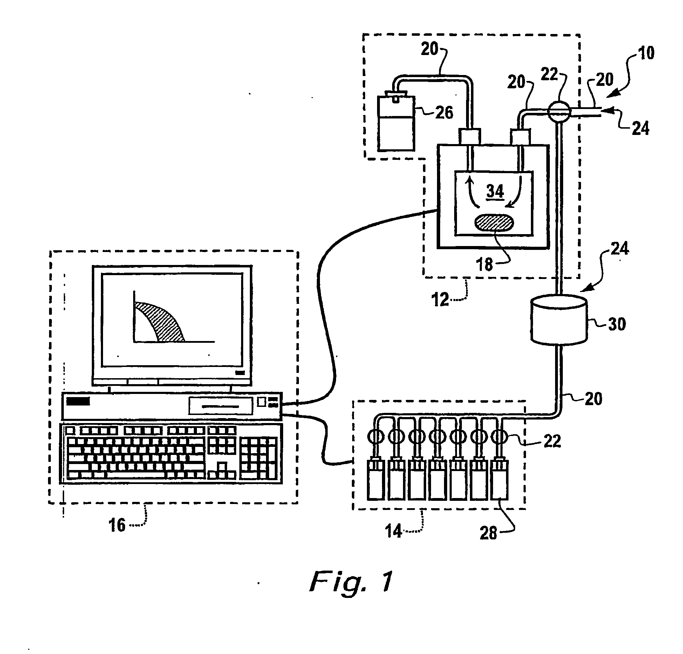

ORAC(o) Apparatus. FIG. 1 is a depiction of an ORAC(o) direct anti-oxidant apparatus 10. The apparatus 10 has, as depicted, three basic components: a detector system 12, a fluidics system 14 and a data processor system 16, which may be interconnected to provide data capture, fluidic and sample control and data processing. The detector system 12 has an oxygen sensor 18, which is in fluid communications with the fluidic system 14 via one or more conduits 20. Fluid flowing through the one or more conduits 20 is controlled using one or more valves 22, which may be manually controlled and/or under the control of the data processor system 16. In operation, a sample 24 enters the fluidic system and is directed into the detector 18, and after data capture is delivered to waste storage 26. The fluidics system 14 may also include one or more solutions 28 that directed into transit through the fluidic system 14 by pumps, by vacuum or by pressure, e.g., pressurized inert gas. The solutions 28 in the fluidics system 14 will generally be premixed or equilibrated, as for use with the present invention may generally include: water, a solvent, a detergent or water:detergent mix, an oxygen radical generator, an oxidation target, etc., and may be mixed at mixing chamber 30 prior to delivery to the oxygen detection chamber 34. The choice of fluidics system will depend on the extent of automatization desired or chosen, as will be known to those of skill in the art. The sample 24 may be pre-mixed with the same solution that is used to calibrate the oxygen sensor 18 or may even be pre-mixed at the mixing chamber 30.

Examples of oxygen detectors 18 for use with the present invention will include any dissolved oxygen sensor that is able to detect dissolved oxygen in the presence of a solvent, water and a detergent. Examples of dissolved oxygen sensors include, e.g., electrochemical, chemiluminescent, surface plasmon resonance, infrared, capacitance coupled, dye-coupled fiber optic or even hyperspectral oxygen sensors. In one specific example, the dissolved oxygen sensor is an YSI 5300A biological oxygen sensor (YSI, USA), a SPREETA sensor (Texas Instruments), a PASCO PS2108 (Pasco, USA), and the like. In one example, the dissolved oxygen sensor has the following specifications: Range of: 0-20 mg/L; Accuracy: $\pm$10% of full scale; Resolution: 0.01 mg/L; Maximum Sample Rate: 20 sps; Default Sample Rate: 2 sps; Response: 98% in 60 seconds; Temperature Range: 0-50°C; Temperature Compensation: 10-40°C; Cathode: Platinum; Anode: Ag/AgCl; Membrane: 1 ml silicon, and may be used in conjunction with the software provided by the manufacturer, e.g., Dissolved Oxygen EZ (Pasco, USA). The system may also include pH, ORP, conductivity or turbidity sensors in fluid communication with the fluidics system

In operation, the ORAC(o) system disclosed herein may be used as follows: a user collects a sample and dissolves it in or with a ORAC(o) solvent kit (dry or liquid). An ORAC(o) sensor, e.g., a hand-held surface plasmon resonance oxygen sensor (see, e.g., Texas Instruments SPREETA sensor) is exposed to one or more calibration standards and then exposed to the user sample. The oxygen sensor is connected to a processor that evaluates the output from the detector surface on the sensor and provides the user with a read-out. The read-out may be displayed on a screen, printed and/or transmitted to a processor, memory and the like. The user sample may be a urine, saliva, tears, mucus secretions, sweat, blood (or blood products), tissue, feces or other biological samples suspected of having oxygen radicals. In one example, the sample is one or more breaths (one or more inhalations and/or exhalations) that are collected by a respirator, e.g., a closed respirator. The values detected by the sensor may even be saved in memory (volatile, semi-permanent or permanent) for future reference or for comparison to past or future values to evaluate the oxidative state of the user.

The basic components of the ORAC(o) assay for antioxidant activity of the present invention take advantage of existing ORAC-like methods and are therefore easily adaptable for use in laboratories without the need for extensive training, if any. Briefly, the ORAC(o) uses an oxygen sensor, for example a blood plasma oxygen sensor or a dissolvable oxygen sensor to measure pro-oxidant activity, e.g., by measuring directly the relative activities of one or more oxygen radical generating molecules in the sample solution and a oxidative quencher (anti-oxidant) as standards. It must be noted that certain agents, e.g., reducing or volatile agents, can lead to the absorption or production of oxygen in solution in the absence of a radical source, e.g. AAPH, can affect the amount of oxygen in the solution. Using the present invention the skilled artisan can differentiate between radical quenching and non-radical quenching activities of samples by, e.g., evaluating the behavior of the sample in solution before the addition of the free radical initiator. It should be noted that both the radical quenching and non-radical quenching activities of the samples tested, as determined by use of the present invention, relate to oxidative state. The relative activities of the oxygen radical generator and the oxygen radical quencher may be titrated and/or measured over time as with the indirect methods ORAC(fl), ORAC(fl-lipo), ORAC($\beta$-PE) and the like.

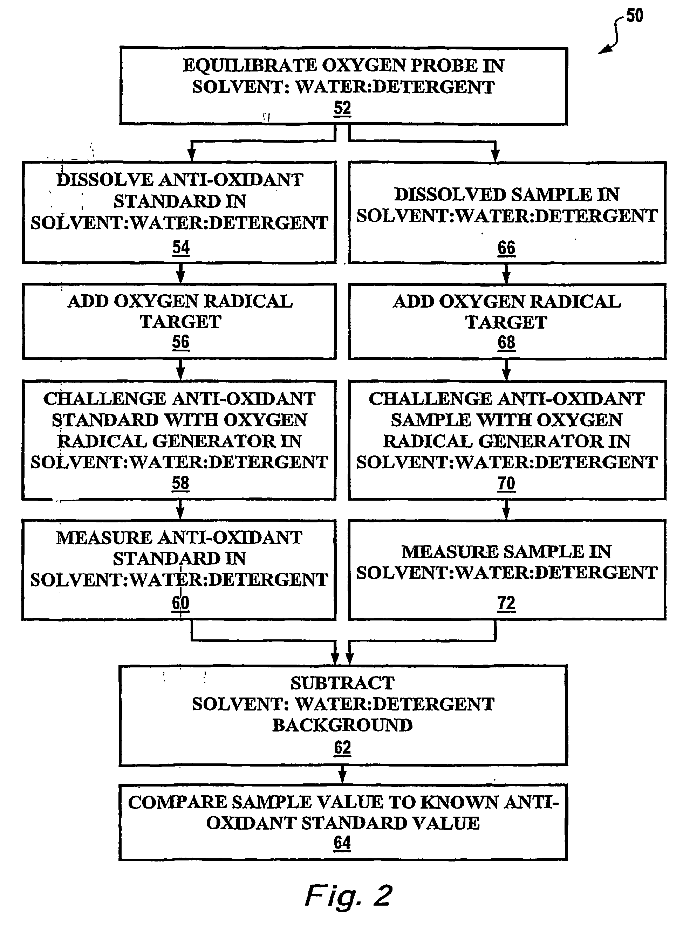

Figure 2 is a flowchart 50 that summarizes the basic steps of the method of the present invention. In step 52, the dissolvable oxygen probe is equilibrated and/or calibrated in the presence of the solvent:water:detergent mixture and a baseline measured. In one examples the solvent:water:detergent is an acetone:water:Tween-20 mixture at a 1:1:1 ratio. In step 54, a baseline determination of anti-oxidant activity serves as the positive control and baseline for comparison of anti-oxidant activity using, e.g., Trolox® as the anti-oxidant. One advantage of Trolox® and related molecules is that these Vitamin E derivatives are more stable from lot to lot, have less lot variation and are synthetic, thereby providing a reliable concentration of anti-oxidant activity. The mixture in step 54 is mixed, in step 56, with an oxygen radical target, e.g., linoleic acid prior to the addition of the oxygen radical generator in step 58. The assay is allowed to run and, in step 60, the area under the curve (AUC) is calculated by measuring the disappearance of dissolved oxygen over time, and the value for the sample stored. In series or parallel, a sample is dissolved in the solvent:water: detergent mixture (step 66) followed by the addition of the oxygen radical target in step 68. Generally, it is common to use the same type of oxygen radical target in steps 56 and 68, and the same type of oxygen radical generator in steps 58 and 70. In step 72, the AUC for the sample is detected, and the value for the sample stored. Now that the standard and the sample AUC have been determined, the values are normalized by subtracting the AUC for the blank. The normalized standard and sample AUC values are then used to compare and calculate the level of anti-oxidant activity in the sample.

The following discussion is used to help illustrate the invention and should not be used limit its scope. The detergent Tween 20 may aid in the dispersion of linoleic acid. Linoleic acid provides double bonds across which oxygen can be absorbed. Trolox® is a synthetic antioxidant used as an internal standard. The values obtained from all samples are related back to those of Trolox®. The oxygen radical generating molecule: 2,2' azobis (2-amidinopropane)dihydrochloride (AAPH reacts) with oxygen to create carbon-centered radicals. The radicals generated by AAPH cause the oxidation of linoleic acid. As a result of the oxidation of linoleic acid, linoleic acid's double bonds become ketones, bonding with oxygen molecules in the carbon-centered radical. The oxygen probe takes measurements of the rate at which the oxygen is being removed from the reaction chamber due to the oxidation of linoleic acid. The azo radical may react directly with linoleic acid, causing the formation of a linoleic acid radical. The linoleic acid radical then reacts with the oxygen present in the reaction chamber to form a ketone. Through either proposed mechanism, oxygen is consumed due to the oxidation of linoleic acid. The antioxidant slows the consumption of oxygen in the reaction chamber by deterring the oxidation of linoleic acid. The calculation of the area under the curve for dissolved oxygen versus time plot yields a measure of the sample's antioxidant capacity as demonstrated by its ability to slow the oxidation of linoleic acid.

The oxygen radical generators. Azo-radical generators are present in the ORAC(o) assay of the present invention at a known concentration to generate radicals for measurements of antioxidant activity. Azo initiators include, for example, 2,2'-azobis[2-(5-methyl-2-imidazolin-2-yl)propane]dihydrochloride, 2,2' azobis (2-amidinopropane)dihydrochloride (AAPH), 2,2'-azobis(2-amidinopropane)[2-(N-stearyl)amidinopropane] dihydrochloride (SA-1), 2,2'-azo(2-(2-imidiazolin-2-yl)-propane)-[2-[2-(4-n-octyl)imidazolin-2-yl]-propane] dihydrochloride (C-8), 2,2'-azobis(4-methoxy-2,4-dimethylvaleronitrile) (MeO-AMVN), 2,2'-azobis(2,4-dimethylvaleronitrile) (AMVN), azo-bis-isobutylnitrile, 2,2'-azobis (2-methylproprionate) (DAMP), and 2,2'-azobis-(2-amidinopropane).

For example, the radical generator 2,2' azobis (2-amidinopropane) dihydrochloride (AAPH) decomposes into molecular nitrogen and two carbon radicals. The carbon radicals combine to produce stable products or react with molecular oxygen to give peroxyl radicals. The half life of AAPH is about 175 hours (37 °C at neutral pH). Therefore, the rate of free radical generation is essentially constant during the first several hours in solution. AAPH is used is often used for lipid peroxidation in aqueous dispersions of fatty acids, as such; it can be used alone or in combination with a lipohilic and/or a lipophobic radical generator. The solvent system disclosed herein allows for use of either or both lipohilic and/or a lipophobic radical generators as the apparatus measures total oxygen in the sample.

Solvent System. The solvent system for the ORAC(o) is a three-part system that includes a solvent, an aqueous phase and a detergent, wherein the solvent is acetone. It thus consits of one-third water, one-third detergent ("one-third" solvent), and the sample in acetone at a concentration of, e.g., 1 mg/mL. Dilutions are then made using the same solvent. The detergent is a nonionic detergent such as TWEEN® (i.e., TWEEN®20), BRIJ®, or TRITON®. Unlike the ORAC(fl), which uses a two component system that uses of one-half acetone and one-half water, the detergents of the ORAC(o) solvent system permit a direct measurement of oxygen from the total sample. One variant is the ORAC(fl-lipo) that uses a randomly methylated β-cyclodextrin.

Uses for the ORAC(o) Assay: The ORAC(o) assay may be used to measure the total antioxidant activity of biological samples, for the evaluation of components for the nutritional supplements of the present disclosure and even for testing and evaluating competitor and/or the final dietary supplement of the present disclosure. For use in the evaluation of biological samples, these may be, e.g., serum, lipid-soluble serum fraction, water-soluble serum fraction, urine, lipid-soluble urine fraction, water-soluble urine fraction, LDL fraction, tissue homogenates, quality control of antioxidant supplements, food products, or preservatives, development of new antioxidant supplements, development of new food products, new preservatives, or new antioxidant therapies, quality control of food manufacturing and processing, assessing antioxidant activity of plants, or monitoring the antioxidant activity of cosmetic products, for example.

EXAMPLE 1: Comparison of Antioxidant Activity Using ORAC(fl) and ORAC(o) Assays.

Ingredients for an antioxidant composition were analyzed for antioxidant activity using a prior art oxygen radical absorption capacity method that measures fluorescence (ORAC(fl)), and using the method of the present invention that measures dissolved oxygen (ORAC(o)). The antioxidant activity of a product is its ability to protect the system from damage caused by peroxyl radicals.

Prior Art Method of Measuring Antioxidant Activity for Comparison. For the ORAC(fl) assay, the method of Ou et al. (Ou, B., Hampsch-Woodill, M. and Prior, R.L., J. Agric. Food Chem. 2001, 49, 4619-4626) was followed. Differences from the Ou procedure as published included the speed of the orbital shaker (Ou, 400 rpm; herein, 280 rpm), and the centrifuge speed (Ou, 14,000 rpm, 15 min; herein, 3200 rpm, 15 min and the length of the assay (Ou, 30 min; herein, 100 min). Fluorescein, sodium salt, was obtained from Aldrich (Milwaukee, WI). For the Ou method, a standard amount of fluorescein is added to an antioxidant product being tested, and the beginning level of fluorescence is measured. A free radical initiator is added, and the time and degree of disappearance of fluorescence are measured. Trolox®, 6-Hydroxy-2,5,7,8-tetramethylchroman-2-carboxylic Acid, is a cell-permeable, water-soluble derivative of vitamin E with potent antioxidant properties. Trolox® prevents peroxynitrite-mediated oxidative stress and apoptosis in rat thymocytes and is a synthetic antioxidant that is consistent from lot-to-lot and is used as the standard and run for comparison with each sample. A blank (control) used in calculations of the ORAC(o) value for each samples may be included in each run. Fluorescence vs. time is plotted. The blank (control) is subtracted from every curve. The net area under the curve of the antioxidant is compared to the net area under the curve of Trolox®. The larger the net area under the curve, the great the antioxidant capacity of the molecules in the sample. All ORAC(fl) analyses were performed on a COBAS FARA II centrifugal analyzer (Roche Diagnostic System Inc., Branchburg, NJ; excitation wavelength = 493 nm and emission filter = 515 nm). Results are given as micromoles of Trolox® equivalents per gram of sample.

Method of Measuring Antioxidant Activity. In operation, the ORAC(o) assay of the present invention was used to evaluate antioxidant compositions, combinations and the like of solutions against a target molecule (linoleic acid) with oxygen present at equilibrium with the air. A free radical initiator is added and the time it takes for oxygen to disappear is measured, yielding the rate of disappearance. Trolox® is used as the standard, and a blank is run as a control. Amount of dissolved oxygen vs. time is plotted allowing direct comparison of net areas under the curve. A detailed method is as follows: Buffers (K2HPO4 (F.W. 174.2), NaH2PO4 (FW 120.0)) were obtained from Sigma (St. Louis, MO). Linoleic acid 99%, Trolox® (6-hydroxy-2,5,7,8-tetramethylchroman-2-carboxylic acid) 97%, TWEEN® 20, and 2,2'-azobis (2-methylpropionamidine) dihydrochloride 97% (AAPH), were obtained from Aldrich (Milwaukee, WI). HPLC grade acetone was obtained from Fisher (Hampton, NH). A YSI 5300A biological oxygen meter was obtained from YSI (Yellow Springs, Ohio) and used according to the manufacturer's specifications.

ORAC(o) Method. The following stock solutions were used. Preparation of phosphate buffer (75 mM, pH 7.4); 750 mM $K_2HPO_4$ (F.W. 174.2); Weigh 65.33g into 500mL of volumetric flask, and add dH20 to the mark. Concentration is 750 mM. Store 750 mM $K_2HPO_4$ stock solution in a refrigerator (2 to 8°C) equipped with a calibrated thermometer. Target temperature is 4°C. 750mM $NaH_2PO_4$ (FW 120.0); Weigh 45.00 g into 500 mL of volumetric flask, and add dH20 to the mark. Concentration is 750 mM. Store 750mM $NaH_2PO_4$ stock solution in a refrigerator (2 to 8°C) equipped with a calibrated thermometer. Target temperature is 4°C. Mix 40ml of 750 mM $K_2BPO_4$ stock solution and 10ml of 750mM $NaH_2PO_4$ stock solution (4 to 1 ratio). This will yield 50 mL of ORAC stock solution buffer. Store stock solution in a refrigerator (2 to 8°C) equipped with a calibrated thermometer. Target temperature is 4°C. Dilute mixture with dH20 (1:9) to make working solution of ORAC buffer, and measure pH. It should be 7.4. Keep covered at room temperature until ready to use.

Tween Preparation. Weigh out 10g of Tween 20, and add 90 g of deionized water. Cover and stir the mixture overniglit. Tween stock solution keeps for one week. Keep on countertop.

Solvent Preparation. Mix 25 mL of acetone, 25 mL of deionized water, and 25 mL of Tween 20. Use this solvent to prepare samples. Before a run is performed: the probe should be inspected for damage paying particular attention to the membrane, replace if needed. Probes should be stored with the tips in deionized water. Only one probe and one reaction chamber should be used for this assay. Run a blank and Trolox® (at 10 $\mu$g/ml or 0.03995 $\mu$mol/ml) every 8 hours.

Samples are to be run at a concentration of 10 $\mu$g/ml. The sample concentration and Trolox® concentration should be the same.

The dilutions are prepared by weighing out 1 mg of sample and adding 1 ml of solvent to perform the initial extraction. Shake for one hour, place 0.5 ml of the initial extraction solution in a glass scintillation vial, and then add 4.5 ml of solvent, making the first dilution. Vortex the first dilution for about 30 seconds. Its concentration is 0.1 mg/ml or 100 ppm. Take 0.5 ml of the first dilution, put it into another glass scintillation vial, and then add 4.5 ml of solvent, vortex for about 30 seconds, to yield a concentration of 0.01 mg/ml or 10 ppm. Take 0.5 ml of the vial sample and put it into another glass scintillation vial, and then add 4.5 ml of solvent, vortex for about 30 seconds, to yield a concentration of 0.01 mg/ml or 10 ppm. The vials are placed in a 4° C refrigerator. In a 37° C water bath allow linoleic acid to thaw, and prepare linoleic acid solution (with minimal light) by adding 0.18 mL Tween stock solution, 0.18 mL buffer, and 0.44mL deionized water to 70.8 mg linoleic acid. Keep solution tube wrapped in foil. Make linoleic acid solution fresh every 8 hours. To prepare the oxygen radical generator, weigh 67.8mg of AAPH, and dissolve in 0.9 mL buffer. Keep AAPH refrigerated, it may be used for up to eight hours. Shake for 1 hour. The Trolox® solution should be kept refrigerated at all times. To begin measuring anti-oxidant activity, add 1.86 mL of buffer into each reaction container, 2.36mL water, to the scintillation vials at 37° C and allow three minutes of stirring while the reaction matrix comes to temperature. Add 0.284 mL of the sample, and place the probe tip into the vessel without touching the reaction solution, and allow one minute of stirring. The measurement are best taken in a light-tight environment.

Following data capture, remove the probe from the chamber, add 0.357mL each of linoleic acid solution, and replace the end of the probe into the chamber immediately. Add 0.357ml of AAPH solution, and place the probe into the solution tube carefully but quickly so that no bubbles are left in the reaction area, and no solution is in the overflow ridge on the probe case. Next, place probe into the solution and take a reading. Capture data and calculate anti-oxidant activity.

Samples were prepared at a concentration of 1mg/mL in "one-third solvent" (also referred to as the ORAC(o) solvent system). A "one-third" solvent is equal parts of water, acetone, and a solution of TWEEN®20 diluted 1:9 with water. Samples were shaken for 1 hour at room temperature on an orbital shaker at 280 rpm. The sample solution was ready for analysis after further dilution (generally to 10 $\mu$g/mL) with "one-third" solvent. "One-third" solvent was also used as the blank.

The ORAC(o) assay was carried out using the YSI 5300A biological oxygen meter. Linoleic acid was prepared by adding 0.18 mL of 75 mM phosphate buffer (pH 7.4), 0.18mL of 10 weight percent TWEEN®20 stock solution, and 0.44 mL of deionized water to 70.8 mg linoleic acid. AAPH was prepared by adding 0.9 mL of buffer to 67.8 mg of AAPH. In the final reaction volume (5.218 mL), linoleic acid (21.59 mM) was used as the target of free radical attack, and AAPH (19.00 mM) was used as a peroxyl radical generator. Trolox® (at 10 $\mu$g/mL) was used as a standard. Readings were taken every second until a zero reading was observed.

The formula for calculating the ORAC(o) value (Oxygen Radical Absorbance Capacity oxygen specific electrode) is:

$$ORAC(o) = \frac{\dfrac{AUC_{SMP}-AUC_{BLNK}}{AUC_{TRLX}-AUC_{BLNK}} \times 1000 \ (mg/gr) \times [TRLX \ (\mu mol/ml)]}{[SMP \ (mg/ml)]}$$

Alternatively, the ORAC(o) may be calculated in milliliters when evaluating a liquid formula. This calculation yields a quantity known as micromoles of Trolox® equivalents per gram of sample. A negative value ORAC(o) reflects less radical quenching activity than obtained with a blank which indicates that a composition is a pro-oxidant, i.e., an agent that promotes oxidation, rather than acting as an antioxidant. An assumption of the ORAC(o) assay is that oxygen is not being absorbed or released by the sample, however, any effect in the oxygen level of the sample can be evaluated and used to compensate into the calculated anti-oxidant value.

Comparison of ORAC(o) and ORAC(fl) Assay Parameters. The advantages of the ORAC(o) as compared to the ORAC(fl) were measured in a direct vs. indirect measurement of antioxidant potential. The ORAC(fl) is an indirect oxygen radical detection method because it relies on the assumption that the fluorescein (target molecule) is the only fluorescent component being measured. However, many antioxidant compounds fluoresce naturally (e.g., blueberries); and combinations of these compounds from radical-radical reactions fluoresce also. Fluorescence from the sample, therefore, can skew the results of an assay based on fluorescence. The ORAC(o) method, on the other hand, is a direct measurement of oxygen uptake, that is, a direct measurement of the disappearance of oxygen into free radicals. This method of measurement of antioxidant capacity is not dependent upon whether the oxygen is attached to water- or lipid-soluble components. A comparison of the assay parameters of the ORAC(fl) and the ORAC(o) methods is provided in Table 1.

**Table 1:** Comparison of Assay Parameters for ORAC(fl) and ORAC(o).

| Assay Parameter During Assay | ORAC(fl) | ORAC(o) |
|---|---|---|
| Temperature | 37°C | 37°C |
| AAPH concentration | $1.28 \times 10^{-2}$ M in phosphate buffer | 19.00 mM in phosphate buffer |
| Target molecule concentration | fluorescein, 43.8 nM in phosphate buffer | 21.59 mM linoleic acid, in mixture of 10% TWEEEN® 20, buffer and water (.18 mL, .18 mL, .44 mL) |
| Sample concentration | initially at 500 mg in 20 mL, supernatants diluted to 10 $\mu$g/mL with buffer | 1 mg/mL initially, diluted to 10$\mu$g/mL |
| Sample solvent | acetone/water, 50/50 v/v dilutions in phosphate buffer | water/acetone/10% by weight TWEEEN® 20, v/v/v ("one-third" solvent) dilutions in "one-third" solvent |
| Buffer for assay, concentration | 75 mM phosphate, pH 7.4 | 75 mM phosphate, pH 7.4 |
| Final assay volume | 400 $\mu$l | 5.218 mL |
| Blank (control) | buffer | "one-third" solvent |
| Standard | 62.5 $\mu$M Trolox® in buffer | Trolox® at 10 $\mu$g/mL (38.755 $\mu$M) in "one-third" solvent |
| Method of measuring anti-oxidant capacity of the sample | fluorescein yields a decrease in fluorescence as it is oxidized by radicals, antioxidant protects and delays decrease | oxygen is taken up by the radical initiator, and by linoleic acid carbon radicals; antioxidant protects and decreases oxygen uptake |

One distinct advantage of the present invention is that the user does not need to learn new techniques or purchase equipment to incorporate the present apparatus and method into their laboratory environment. For example, when measuring sample saturation, the present invention uses many of the same buffers, conditions, extraction and/or separation steps as in the well-established indirect measurement system developed by, e.g., Ou, et al. Ou, et al., prepared samples in acetone/water (50:50, v/v) at a concentration of 500 mg in 20 mL, rotated them on a rotary shaker at 400 rpm for 1 hour, centrifuged them at 14,000 rpm for 15 minutes, and diluted them with 75 mM potassium phosphate buffer at pH 7.4 (Ou, et al., Development and Validation of Oxygen Radical Absorbance Capacity Assay for Lipophilic Antioxidants Using Randomly Methylated-β-Cyclodextrin as the Solubility Enhancer, J. Agric. Food Chem. 2002, 50, 1815-1821). The present inventors found that much of a test sample would not go into solution under the Ou conditions (ORAC(fl) conditions), the more soluble components displace the less soluble components. Consequently, only that portion of the sample that was solubilized under the ORAC(fl) conditions was included in the ORAC(fl) sample and measurement. Therefore, a supernatant solution made according to the ORAC(fl) method for analysis is unlikely to be representative of the total anti-oxidant activity of the actual sample. Since a solution of a sample in the ORAC(fl) buffer does not always reflect the contents of the sample, results can be skewed using the ORAC(fl).

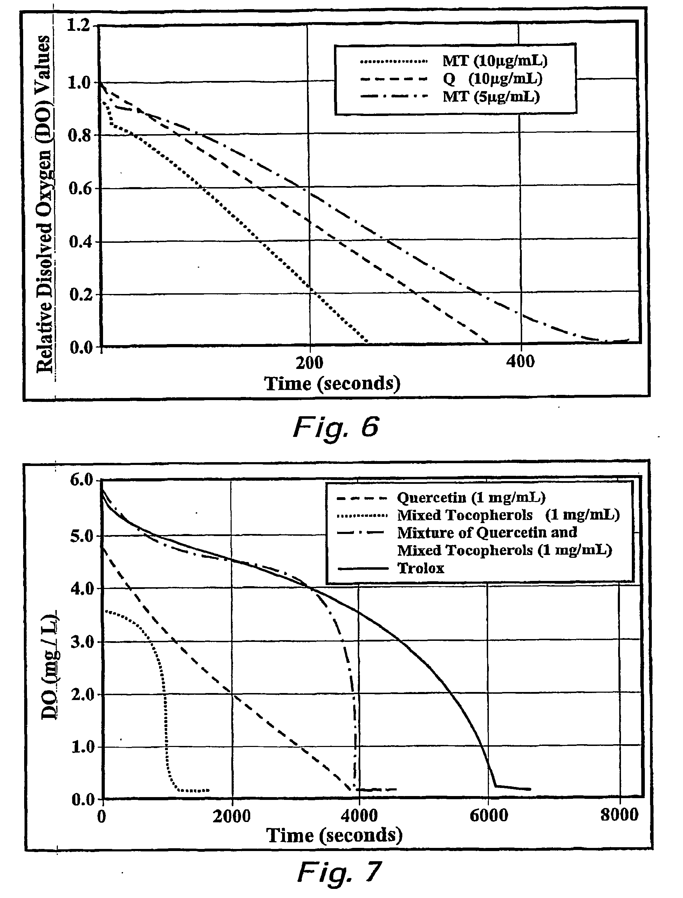

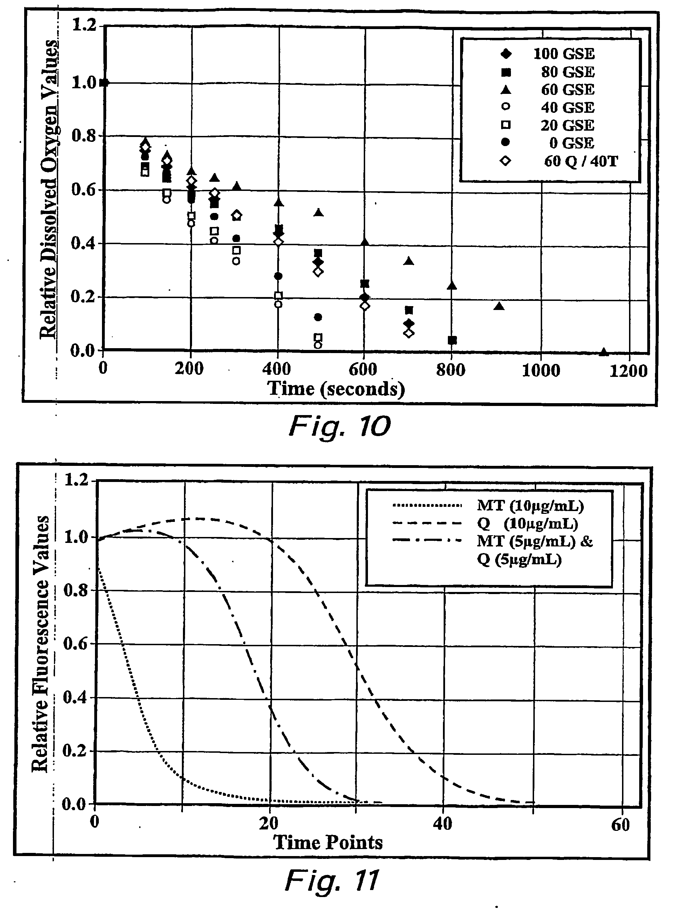

The problem with sample dissolution was recognized and a lack of contribution of mixed tocopherols to an antioxidant measurement carried out on a mixture of quercetin and mixed tocopherols when using the ORAC(fl) was observed. As shown in FIG. 7, using the ORAC(o) system, it was found that the antioxidant effect of the combination of quercetin at 5 μg/mL and mixed tocopherols at 5 μg/mL as compared to each ingredient separately at 10 μg/mL. In contrast, the indirect ORAC(fl) system shows no further effect from the same combination beyond the value for quercetin alone (see FIG. 11). Lack of activity from such a combination with mixed tocopherols and saturation problems are absent from the ORAC(o) method since the concentration of sample is more dilute and since the solvent for the sample contains acetone, water, and a detergent for solubilizing all components of a sample. Presence of TWEE®20 in the solvent for the sample accounted for detection of activity from mixed tocopherols. As a control for Trolox® activity, FIG. 7 shows the quercetin at 5 μg/mL and mixed tocopherols at 5 μg/mL as compared to each ingredient separately at 10 μg/mL but also includes the Trolox® control and demonstrates the ability of ORAC(o) to detect the level of oxygen radicals remaining in the sample over time.

The ORAC(o) provides a significant savings as compared to the more expensive ORAC(fl) (automated circa $250,000; non-automated circa $50,000). In contrast, the ORAC(o) apparatus disclosed herein takes advantage of off-the-shelf oxygen sensor systems that may be easily miniaturized and/or automated that may be developed for sale to doctors offices and even home use at a fraction of the costs of large fluorimeter detection systems. Validation, as conducted by the Association of Official Analytical Chemists (AOAC) guidelines for single laboratory validation, provided results in Tables 2, 3, and 4.

**Table 2.** 5 Day Trial for Precision (Repeatability)

|  | Area under Curve |
|---|---|
| Day 1 | 202.655447939563 |
| Day 2 | 212.901122995373 |
| Day 3 | 187.01466464995 |
| Day 4 | 202.856617129109 |
| Day 5 (2nd Analyst) | 213.124092629655 |
|  |  |
| 5 Day Mean | 203.710389068730 |
|  |  |
| Standard Deviation | 10.649840914564 |
| Relative Standard Deviation | 5.23% |
|  |  |
| HORRAT | 1.981060606061 |

The values for each day are an average of 3 runs of the quercetin sample at a concentration of 10μg/mL. The HORRAT value is the ratio between observed $RSD_R$ values and the $RSD_R$ values predicted by the Horwitz equation known to those of skill in the art, and is regarded as an indication of the acceptability of a method with respect to its precision. In a single laboratory performance study, a series of HORRAT ratios between 0.5 and 2.0 indicate acceptable precision of a method. The HORRAT value for the 5-day trial is 1.98. A determination of the analytical range as a linear range is provided in Table 3.

**Table 3.** Determination of Analytical Range

| Sample (µg/ml) | Area Under Curve |
|---|---|
|  |  |
| 1 | 124.567114093960 |
| 10 | 209.388111888112 |
| 100 | 693.611111111111 |
| 500 | 2327.96518987343 |
| 1000 | 2870.09540636041 |
|  |  |
| y = 2.8335x + 332.16; $R^2$ = 0.9231 for 1-1000 plot<br>y = 4.3353x + 176.66; $R^2$ = 0.9967 for 0-500 plot | |

Linear integrity appears to decline as the sample concentration nears 1000 µg/mL. With the determination of the analytical range as a linear range, the value of the area under the curve of variable concentrations up to 500 µg/mL is determined. Table 4 provides results for precision of single-day results.

**Table 4.** Single Day Trial for Precision (Repeatability)

|  | Area under curve |
|---|---|
| Run 1 | 207.369402985075 |
| Run 2 | 198.18118466899 |
| Run 3 | 205.035211267606 |
| Run 4 | 201.586572438162 |
| Run 5 | 202.110714285714 |
|  |  |
| 5 Run Mean | 202.856617129109 |
| Standard Deviation | 3.505016471434 |
| Relative Standard Deviation | 1.73% |
|  |  |
| HORRAT | 0.654480870834 |

The single day precision trial involved 5 separate runs of the quercetin sample at a concentration of 10µg/mL. The HORRAT value of Table 4 for the ORAC (o) method is 0.65448.

The ORAC(o) assay was used to optimize ratios of the ingredients in an antioxidant composition as set forth in Example 2. One embodiment of the composition has weight ratios of quercetin, 49.18%; mixed tocopherols, 32.79%; grape skin extract, 9.84%; green tea extract, 6.56%; and bush plum, 1.64% gave an antioxidant value using the ORAC (o) of 17,254 micromoles Trolox® equivalents per gram. The same composition gave an antioxidant value using the ORAC(fl) of 5,281 micromoles Trolox® equivalents per gram. These data demonstrate that the ORAC(o) and ORAC(fl) methods of measuring antioxidant activity differ in the results obtained due to the limitations of the indirect ORAC(fl) method.

EXAMPLE 2: A Synergistic Antioxidant Composition.

According to the dietary supplement of the present disclosure, five ingredients were combined into an antioxidant composition, each of which is prominent in the diet of long-lived peoples from regions around the world. The present inventors selected ingredients based on a comprehensive search of the diets in areas known for longevity. The present inventors recognized that the diets in these regions were rich in flavonols and tocopherols. It was further recognized by the inventors that certain natural compounds interact favorably with molecules that reactivate their anti-oxidant activity, e.g., vitamin C. In fact, extensive research supports the role of Vitamin C in the regeneration of, e.g., $\alpha$-tocopherol (Pizzorno and Murray, Textbook of Natural Medicine, 1999, 2nd Ed. New York, Churchill Livingston).

Based on this research, the inventors combined isolated and purified extracts from natural and other sources that include high percentages of bioavailable compounds from the various regions of long-lived peoples into one formulation. In one example, the following basic ingredients were selected: flavonols, mixed tocopherols, grape skin extract, green tea extract, or bush plum are prominent in the diet of peoples of the Andean village of Vilcabamba in Ecuador, the land of Huza in the Karakoram Range in Kashmir, or in Abkhazia in the Georgian State of the former USSR, for example, as cited in Leaf A., Launois J. "A Scientist Visits Some of the World's Oldest People," National Geographic. 1973 January; of the Italian island of Sardinia (Koenig R. "Sardinia's Mysterious Male Methuselahs," Science. 2001, March 16), and of Australia.

The compositions of the present disclosure demonstrated a synergistic antioxidant activity. Not wanting to be bound by theory, the various ingredients of the antioxidant composition have activity for protecting intracellular cytosol, cellular membranes, and extracellular fluid such that the body is protected throughout. The bush plum component is high, for example, in natural vitamin C content, which can get into the cell, is hydrophilic, and is available for protecting the cytosol; the grape skin extract and green tea extract are hydrophilic, cannot enter the cell and are available for protecting extracellular fluid; and mixed tocopherols are lipophilic, and together with flavonols (e.g., quercetin), which are both hydrophilic and lipophilic, protect membranes. Furthermore, any fiber that is included in the diet, or even in the dietary supplement itself, may provide adequate vehicle for elimination.

Components of the Synergistic Antioxidant Composition. With the availability of the ORAC(o) apparatus and the methods of the present invention, the present inventors were able to measure the total antioxidant activity of combination of both lipophilic and lipophobic anti-oxidants and other agents that aid in potentiating the anti-oxidant activity of these agents, e.g., vitamin-C and the like. Using the ORAC(o) system, an antioxidant composition having synergistic activity was developed that includes flavonoids, e.g., quercetin, a mixture of at least two forms of vitamin E, and optionally, grape skin extract, green tea extract and Australian bush plum. The synergism is particularly observed in a weight ratio of quercetin to the mixture of vitamin E forms of 40/60 to 90/10%. One embodiment of the composition includes the following weight ratios: quercetin, 49.18%; mixed tocopherols, 32.79%; grape skin extract, 9.84%; green tea extract, 6.56%; and bush plum, 1.64%.

FIG. 6 is a graph that shows the results of an ORAC(o) assay comparing quercetin, mixed tocopherols, and mixture of tocopherols and quercetin and the synthetic anti-oxidant standard: Trolox® (Hoffman-La Roche).

Flavonoids such as Quercetin. The flavonoid of the composition can be a flavone, a flavonol, an isoflavone, an isoflavonol, an analogue thereof, a pharmaceutically acceptable salt thereof, or a mixture thereof. Examples of a flavonol include quercetin, kaempferol, and myricetin. The particular flavonoid or flavonoid analogue or salt included in the composition is determined by running an ORAC(o) antioxidant determination. An activity within 80% percent of that of quercetin is contemplated to provide an analogue. Reference to a flavonoid, in particular, quercetin, also is intended to refer to the aglycone or a glycoside thereof where the sugar is arabinose, rhamnose, galactose or glucose, for example. The rhamnose glycoside of quercetin is known as rutin or quercetrin, and the rhamnose glycoside of myricetin is known as myricitrin. Analogues of quercetin include those compounds which comprise a substituting group other than an -OH group at one or more of the positions 3, 5, 7, 3', and 4'. Other substituting groups include: alkyl less than 5 carbon atoms, acetyl, sulphyl, or malonyl. For analogues of quercetin, only one or two of the positions are substituted with anything other than -OH groups.

Flavonoids such as quercetin are readily synthesized *in vitro.* However, flavonoids (including quercetin) are present and may be isolated and purified from, e.g., naturally occurring foodstuffs, in particular, fruits and vegetables, such as apples, pears, grapes, onions, red wine, bell peppers, red currants, black currants, lemons, cherries, cranberries, gooseberries, tomatoes, olives, radishes, kohlrabi, horseradish, potatoes, and asparagus. Quercetin may be obtained from Pharmline (Florida, NY).

Bush Plum: The Australian bush plum (*Terminalia ferdinandiana*) contains about 5% vitamin C and a variety of ingredients as demonstrated by an HPLC chromatogram (data not shown). These ingredients are believed to include flavones and flavonoids. The HPLC chromatogram is from a reverse phase $C_{18}$ column using a stepped gradient of 0.1% trifluoroacetic acid and 100% methanol, at a flow rate of 1mL/min using a sample of methanol and water-extracted bush plum powder. The conditions were developed to separate flavonoids and to separate vitamin C. The absorbance was measured at 245 nm.

Pulp and skin of a bush plum are removed from the seed of the fruit and made into a slurry in water. The slurry is freeze-dried and ground. For antioxidant compositions herein, the freeze-dried material is weighed in the desired amount. Bush plum is present in the composition of the present disclosure in an amount of from 0% to 87.9%, or in another embodiment, about 2% by weight.

Grape skin extract. Grape skin extract is made from grape skins, and contains 30-82% polyphenols and may be obtained from Polyphenolics, Madera, CA; Hunan Kinglong, Bio-Resource Co. Ltd, Changsha Economic Development Zone, China; or from Pharmline, Florida, NY.

Green tea extract. Green tea extract is an extract from the leaves of *Camellia sinensis*, contains 35-95% polyphenols, and may be obtained from Amax NutraSource Inc., Eugene, OR; Blue California, Rancho Santa Margarita, CA; or from PL Thomas & Co., Morristown, N.J.

Other Ingredients. The antioxidant compositions of the present disclosure may include one or more, non-toxic, pharmaceutically acceptable carrier such as lactose, starch, sucrose, glucose, methyl cellulose, magnesium stearate, dicalcium phosphate, calcium sulfate, mannitol, sorbitol, cyclodextrin, cyclodextrin derivatives, or the like. Using these one or more carriers capsule or tablets may be easily formulated and can be made easy to swallow or chew. Tablets may contain suitable carriers, binders, lubricants, diluents, disintegrating agents, coloring agents, flavoring agents, flow-inducing agents, or melting agents. A tablet may be made by compression or molding, optionally with one or more additional ingredients. Compressed tablets may be prepared by compressing the active ingredient in a free flowing form (e.g., powder, granules) optionally mixed with a binder (e.g., gelatin, hydroxypropylmethylcellulose), lubricant, inert diluent, preservative, disintegrant (e.g., sodium starch glycolate, cross-linked carboxymethyl cellulose) surface-active or dispersing agent. Suitable binders include starch, gelatin, natural sugars such as glucose or beta-lactose, corn sweeteners, natural and synthetic gums such as acacia, tragacanth, or sodium alginate, carboxymethylcellulose, polyethylene glycol, waxes, or the like. Lubricants used in these dosage forms include sodium oleate, sodium stearate, magnesium stearate, sodium benzoate, sodium acetate, sodium chloride, or the like. Disintegrators include, for example, starch, methyl cellulose, agar, bentonite, xanthan gum, or the like. Molded tablets may be made by molding in a suitable machine a mixture of the powdered active ingredient moistened with an inert liquid diluent.

Capsules or tablets may optionally be coated or scored and may be formulated so as to provide slow- or controlled-release of the antioxidant composition. Timed-release compositions for controlled release of agents generally contain agent particles mixed with or coated with a material that is resistant to enteric degradation or disintegration for a selected period of time. Release of the agent may occur by leeching, erosion, rupture, diffusion or similar actions.

Carriers may promote antioxidant stability as well as providing time release. A mixture of plant carbohydrates termed AMBROTOSE® Phyto Formula may be combined with the antioxidant composition. Such a combination extends the shelf life of the antioxidant composition and provides for a time release form. AMBROTOSE® Phyto Formula contains, in a weight/weight ratio of about 30/30/20/19/1, gum Arabic(acacia), xanthan gum, gum tragacanth, gum ghatti, (which may be obtained from TicGum) and an aloe vera gel extract (e.g., inner leaf gel, Carrington Labs, Irving, TX, MANAPOL® powder or similar product). The AMBROTOSE® Phyto Formula is blended with the antioxidant composition of the present disclosure in a weight ratio of 2:1 to 1:2. In another embodiment, AMBROTOSE®. Phyto Formula is blended with the antioxidant composition in a weight ratio of 2:1.

Capsules or tablets may contain further plant components in weight percentages less than about 0.1 % to 90% depending on the specific formulation. In the case of carriers, the carriers themselves will generally have no effect on the nutritional significance to the composition, however, these carriers may have a significant in the timing, location and release profile of the release of the nutritionally effective amounts of the present disclosure. For example, one or more of the anti-oxidants of the present disclosure may be released in one or more boluses so as to spike the anti-oxidant levels as detected in, e.g., blood or urine, in a series of release events. In another embodiment, the anti-oxidants may be released somewhat evenly, or may even be provided as a gradient with spikes in blood or urine levels. In fact, the present disclosure may even include release of the lipophobic and the lipophilic anti-oxidants at different times and or locations during digestion. For example, one anti-oxidant may be provided in an effervescent carrier for immediate release, while a different anti-oxidant is provided for release by, e.g., stomach acid or in the intestinal tract.

Formulation Processes. A process of formulating a roller compacted antioxidant composition comprises blending AMBROTOSE® Phyto Formula with the antioxidant composition set forth herein. The resultant blend is transferred to a roller compactor and compacted between rollers to form a compact. The pressure imparted on the blend enhances the physical adhesion between the ingredients. The compact is subsequently milled to form a granulation. A granulation is then formed into the desired dosage form, such as capsules or tablets. In one example, a Fitzpatrick Chilsonator Model 4LX10D roller compactor may be used with rolls that are notched across the face and perpendicular to the rotation, having a fixed force of 10 ton, and a Fitzmill screen of about 0.093. The roller compaction device may have variable rotation speed, force application, and gap width capabilities, for example, a Gerteis Polygran dry roller compactor system (Gerteis, Germany). The roller compactor functions by uniformly applying pressure on a blend by passing the blend between two counter-rotating rollers. The pressure imparted on the blend by the rollers compresses the blend into a compact, such as a sheet or ribbon, which is typically milled to produce granules. Alternatively, granulation may be achieved by slugging, milling or sieving as may be required. Granules having a #20-80 mesh are selected.

A longer shelf life of the roller compacted combination of the antioxidant composition with AMBROTOSE® Phyto Formula is believed due to the reduction in the amount of surface area of the antioxidant composition exposed to oxygen.

The roller compacted combination also eliminates the need for excipient fillers in the capsule or tablet-making process. Additional benefits of a combination of AMBROTOSE® Phyto Formula with the antioxidant composition set forth herein include: provision of non-soluble fiber which may serve as a sink for unpaired electrons in the gut, and provision of monosaccharides for correct structure of cellular glycoforms responsible for cell-mediated communication in repair of cells damaged by free radicals.

Dosage. Useful dosage formulations for administration of the compositions include capsules or tablets of 100, 200, 300,400, 500, 600, 700, 800, 900 or 1000 mg of antioxidant composition. In one embodiment, no fillers, carriers, or stabilizers are added to the composition. In another embodiment, AMBROTOSE® Phyto Formula is blended with the antioxidant composition of the present disclosure in a weight ratio of 2:1, 1:1, or 1:2. In another embodiment, AMBROTOSE® Phyto Formula is blended with the antioxidant composition in a weight ratio of 2:1. In another embodiment, a capsule or tablet provides 500 mg of antioxidant composition blended with AMBROTOSE® Phyto Formula. For this embodiment, two tablets or capsules may be taken per day. Appropriate coatings may be applied to increase palatability or delay absorption.

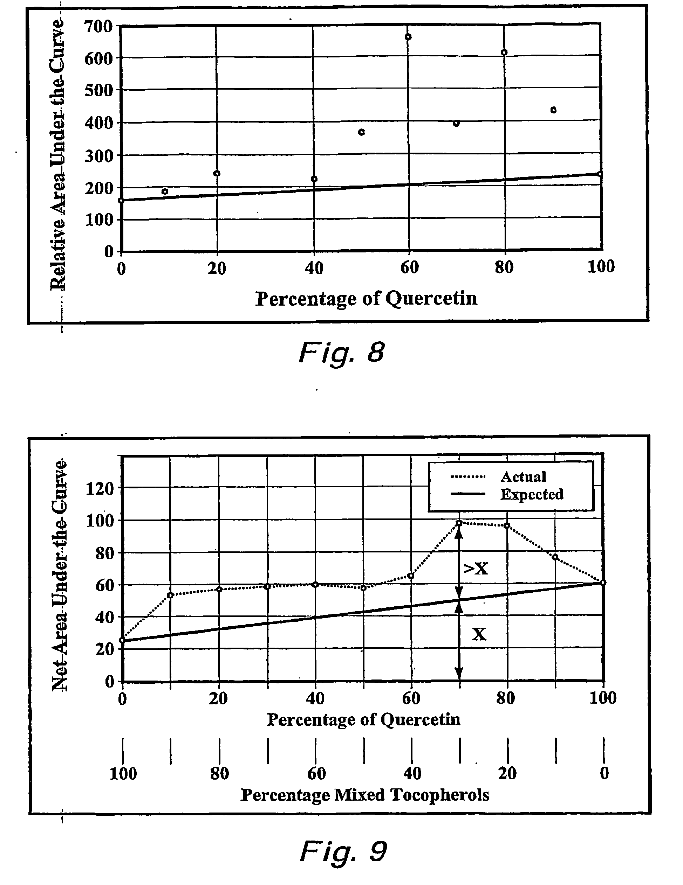

FIG. 8 and FIG. 9, show the net area under the curve (AUC) results for varying ratios of quercetin and mixed tocopherols using the ORAC(o) method to measure antioxidant capacity. The total concentration of sample (Q + MT) is 10 $\mu$g/mL for each percentage assayed. For example, at 0% quercetin, the concentration of mixed tocopherols is 10 $\mu$g/mL, and there is no quercetin in the sample. At 10% quercetin, the sample has 1 $\mu$g/mL of quercetin and 9 $\mu$g/mL of mixed tocopherols. At 20% quercetin, the sample has 2 $\mu$g/mL of quercetin and 8 $\mu$g/mL of mixed tocopherols. The synergy is readily observed between 40 and 100% quercetin, and most readily observed from 40 to 90% quercetin. The straight line represents the additive effect, i.e., at 10% quercetin, the line represents the sum of 90% of the activity of mixed tocopherols alone and 10% of the activity of quercetin alone. A synergistic effect is found above this line.

The primary anti-oxidant ingredients of the composition (flavonoids, as represented by quercetin, and a mixture of at least two forms of vitamin E) comprise from 12.1% to 100%, 30% to 85%, or in another embodiment, about 82% by weight of the five ingredients. In one embodiment, the amount of quercetin is 49.18%, and the amount of vitamin E forms is 32.79% of the total weight of the five ingredients.

The secondary ingredients (or potentiators) of the composition (grape skin extract, green tea extract, and bush plum) comprise from 0% to 87.9%, 15% to 70%, or about 18% by weight of the five ingredients. As shown in FIG. 10, the optimal ratio of grape skin extract and green tea extract was determined in the presence of 49.18% quercetin, 32.79% mixed tocopherols, and 1.64% bush plum. The optimal ratio of grape skin extract to green tea extract is 60/40 to 80/20. In one embodiment, the grape skin extract is 9.84% and the green tea extract is 6.56 % of the total weight of the antioxidant composition. Bush plum (*Terminalia ferdinandiana*) is provided as an ingredient of the composition in an amount from between about 0% to 87.9%, or in another embodiment of about 2%. In the embodiment of FIG. 10, the bush plum is 1.64 % of the composition.

The ORAC(o) assay shows that an antioxidant blend having the following weight ratios: quercetin, 49.18%; mixed tocopherols, 32.79%; grape skin extract, 9.84%; green tea extract, 6.56%; and bush plum, 1.64% has an antioxidant activity of 17,254 micromoles Trolox® equivalents per gram.

Stability of AMBROTOSE AO™. AMBROTOSE AO™, a blend of AMBROTOSE® (Mannatech, USA) and the antioxidant formulation of the present disclosure at a 2:1 weight ratio, has been shown to maintain its activity under accelerated stability conditions (40°C at 75% relative humidity) equating to a shelf-life of roughly six months.

Validation of the ORAC(o) assay as compared to the ORAC(fl) assay. FIG. 11 is a graph of ORAC(fl) results that shown no contribution to AO activity by $\alpha$-tocopherol when mixed at different ratios with quercetin. The graph shows a linear increase in AUC that is directly proportional to increase in quercetin concentration in the ORAC(fl) assay in the standard acetone:water solution. The lack of contribution to AO activity may be due to the failure of the $\alpha$-tocopherol to dissolve in acetone:water.

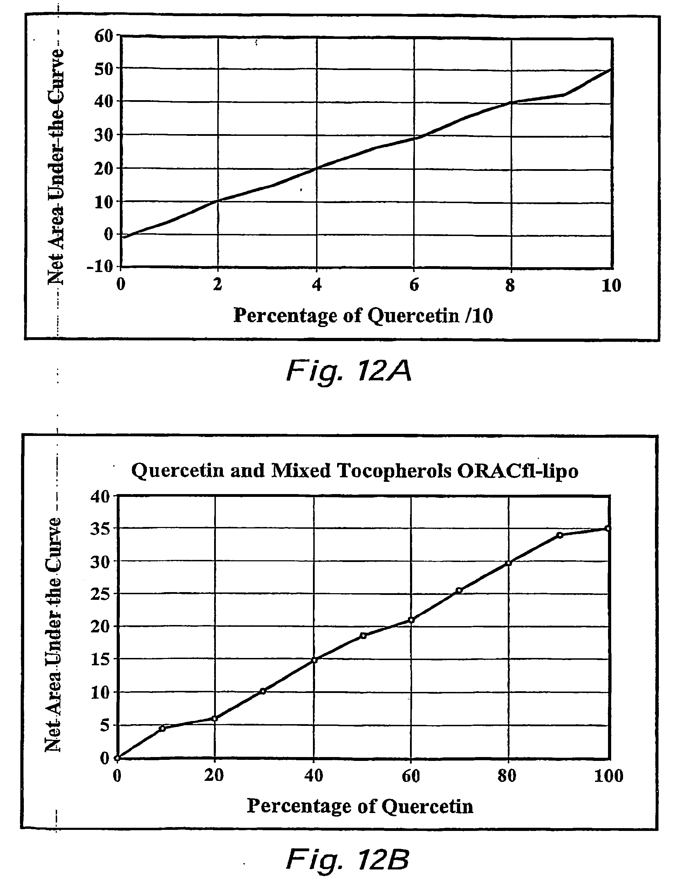

FIGS. 12A and 12B are graphs of ORAC(fl-lipo) results using mixed-tocopherol and the relative AUC results using the solvent/water/detergent solution to dissolve the sample and detect both lipohilic and lipophobic anti-oxidant capacity. In FIG. 11 (above) it was shown that no statistically significant activity could be detected for $\alpha$-tocopherol using the standard ORAC(fl) method for detecting AO contribution of lipophilic AOs. As shown in FIG. 12A, the ORAC(fl-lipo) AUC for $\alpha$-tocopherol was compared between the published ORAC(fl-lipo) method (acetone:water) and an acetone:water:Tween-20 and a large increase in the AUC of the ORAC(fl-lipo) assay was detected. FIG. 12B demonstrates that there is no synergy detected using the known ORAC(fl-lipo) method when combining quercetin and mixed-tocopherols as the results follow a straight line through the abscissa, which indicates lack of synergy using this assay.

Obtaining these results with known assays are not surprising based on a search of the literature and the methods recommended therein. Current standards for the evaluation of so called "High-ORAC Foods" (see www.ars.usda.gov) indicate that the standard is to measure the anti-oxidant potential of lipophilic apart and separate from lipophobic anti-oxidants. The present invention eliminates the need for this dichotomy, a dichotomy that likely results in the disconnect researchers have observed when relating test-tube ORAC values to the effect on serum ORAC values. When measuring the anti-oxidant effects in serum as compared to an ORAC (fl) evaluation, the Agricultural Research Service of the USDA found that, e.g., spinach scored lower than strawberries in the test-tube ORAC assay, but spinach had a greater effect on serum ORAC values than strawberries. Using the present invention and the compositions described herein, the present inventors have remedied the disconnect by developing a system that allows interaction among antioxidant with varying solubilities, which better reflects the activity of antioxidants within the human body. The indirect ORAC(fl) and/or ORAC (fl-lipo) methods fail to measure total anti-oxidant activity and fail to detect the synergistic effects of certain foods.

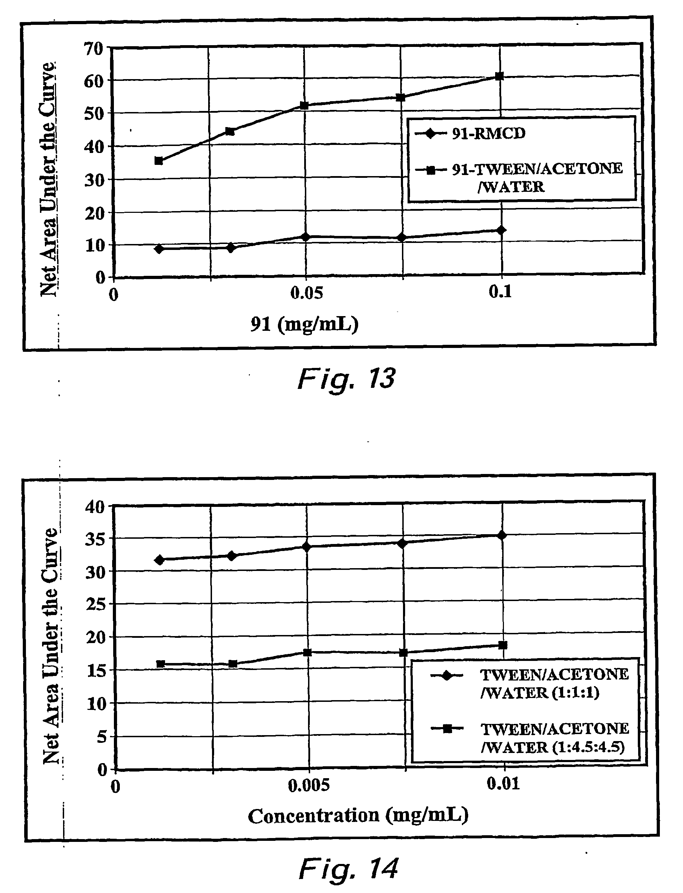

FIG. 13 is a graph of ORAC(fl) AUC results using $\alpha$-tocopherol with a variation in the amount of detergent used to show its effect on AUC measurements. In this assay, the one third solvent was used in its full one third and in a mixture with a smaller amount of Tween-20. FIG. 14 is a graph of an ORAC(fl) assay measuring the anti-oxidant activity measured for a fixed ratio of quercetin:$\alpha$-tocopherol ratio dissolved in two different rations of solvent:water to detergent mixtures. The results in FIG. 13 and FIG. 14, show that the detergent (Tween-20) did account for increase in AUC and therefore shows that detection of ORAC(fl) activity is increased as compared to the standard acetone:water mixture.

EXAMPLE 3. ORAC(o) Detection of Anti-oxidant Synergy and Potentiation.

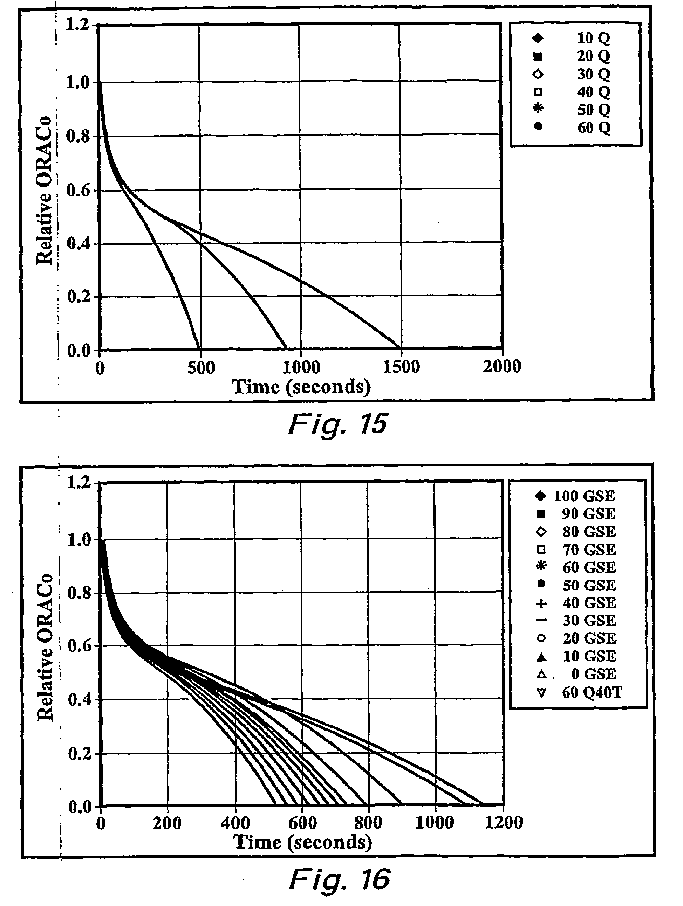

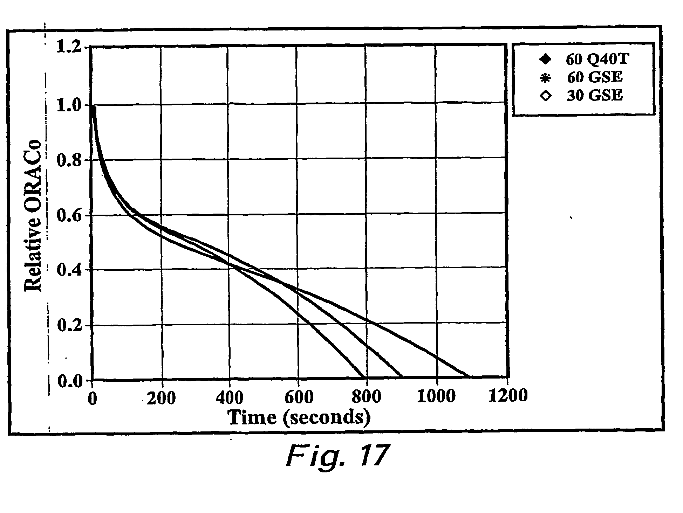

To show that the ORAC(o) assay was able to detect both lipophilic and lipophobic anti-oxidant activity, a series of studies were conducted to show the effect of specific combinations of known concentrations of lipophilic and lipophobic anti-oxidant and the potentiation of the same with the addition of, e.g., grape seed extract and green tea extract as compared to grape seed extract and green tea extract alone. FIG. 15 is a graph showing the ORAC(o) values obtained with different ratios of quercetin and mixed tocopherols. FIG. 16 is a graph showing the ORAC(o) values for different ration of grape seed extract (GES) and green tea extract (GTE). After the determination of the maximum ratio for anti-oxidant activity for GSE and GTE, the two were combined with the optimized ratio for quercetin and mixed tocopherols. FIG. 17 is a graph that shows the ORAC(o) values of the combination of the maximum quercetin:mixed tocopherol and the grape seed extract and green tea extract ratios. It was found that the combined quercetin and mixed tocopherols, GSE, GTE maximized the anti-oxidant potential of the supplement.

EXAMPLE 4: Antioxidant Effect of AMBROTOSE AO™ in a Small Number of Healthy Individuals, Open Label Study, Non-Placebo controlled.

An antioxidant is defined as any substance that can delay or prevent the oxidation of biological substrates. Diets rich in fruits and vegetables that contain antioxidants have been associated with decreased incidence of pathological conditions thought to result from oxidative stress. However, supplementation with isolated antioxidant compounds has often proven ineffective in improving health, in some cases, and dangerous in others.[1,3,4] It has been suggested that the antioxidant benefits of a high intake of fruits and vegetables may not result exclusively from single antioxidants but rather from a concerted action of a combination of different antioxidants present in these foods.[5,6] This focus on single antioxidants is a possible explanation as to why scientists have yet to duplicate the protective effects of an antioxidant-rich diet using nutritional supplementation.[7]

Recent studies have suggested that glyconutritional (GN) supplements, nutritional supplements providing sugars that support cellular communication and immune function, have antioxidant properties both *in vitro* and *in vivo.* Rat liver cells grown in culture medium containing GN supplements showed higher levels of reduced glutathione relative to controls, thereby demonstrating increased antioxidant protection.[8]

A pilot clinical study demonstrated a reduction in biomarkers of oxidative stress and an increase in biomarkers of oxidative defense in people consuming GN supplements. Significant increases were observed in total iron-binding capacity and folic acid, and a significant decrease was observed in copper/ceruloplasmin ratio within 76 days following the daily addition of 2 gr GN supplement to the normal diet. Trends were also observed which suggested a decrease in serum alkenals, homocysteine, 8-hydroxydeoxyguanosine (8-OHdG) and total cholesterol, and an increase in oxygen radical absorbance capacity ($ORAC_{\beta-PE}$).[9]

Recent studies have demonstrated that a wide range of nutrients (some known, others not yet identified) account for the antioxidant activity of fruits and vegetables.[10,11,12,13,14] Some of the known beneficial antioxidants include polyphenols, and vitamins C and E.[6] Certain foods and beverages, including grapes (and red wine), green tea, and the Australian bush plum (*Terminalia ferdinandiana*), contain relatively high levels of such antioxidants.[6,15,16]

This study measured the antioxidant protection provided by a novel nutritional supplement that combined a GN supplement with a blend derived from green tea, grapes, mixed tocopherols, quercetin and the Australian bush plum.

Methods: Blood samples were collected by Cover-Tek, Inc., Dallas, TX. All blood and urine samples were analyzed by Genox Corporation, Baltimore, MD, and all statistical analyses were performed by Decision Analyst, Arlington, TX.

A number of methods for testing the antioxidant potential of samples have been published.[17] The test used in this study was the measurement of whole serum $ORAC_{\beta-PE}$. This method uses $\beta$-phycoerythrin, a fluorescent probe, to determine the antioxidant scavenging capacity of serum samples, specifically against peroxyl radicals, when compared to a known standard, Trolox®. The results are expressed in micromoles Trolox Equivalents (TE) per gram.[18] Other standardized analytical techniques used in the study were the measurement of urine lipid hydroperoxides and alkenals, which relate indirectly to the amount of free radical damage to lipids, and urine 8-OHdG, which relates indirectly to DNA damage.[19,20,21]

The antioxidant nutritional supplement used in this study, Ambrotose AO™, was developed using an *in vitro* evaluation of ingredients: quercetin, mixed tocopherols, grape extract, green tea extract, the Australian plum. The *in vitro* antioxidant values of each ingredient were determined using the standard $ORAC_{fl}$ method (which uses a different fluorescent probe, fluorescein). The antioxidant values of the ingredient mixtures were determined using a newly developed method that measures dissolved oxygen directly, the $ORAC_o$. Since lipid- and water-soluble antioxidants work in concert *in vivo*, the $ORAC_o$, which simultaneously measures the contributions and interactions of lipid- and water-soluble compounds, is an improvement over fluorescence-based methods ($ORAC_{fl}$, $ORAC_{fl-lipo}$ and $ORAC_{\beta-PE}$). These methods, at best, measure the activities of lipid- or water-soluble compounds alone. $ORAC_o$ therefore provides a more accurate determination of the total *in vitro* antioxidant activity of a blend of both water- and lipid-soluble ingredients. The water and lipid-soluble ingredients were combined and evaluated with the $ORAC_o$ to establish maximal synergy and the optimal *in vitro* $ORAC_o$ value of the blend.*

The subsequent blend was roller-compacted with Ambrotose® complex at a ratio of 1:2 to create Ambrotose AO™. Many of the natural gums in Ambrotose® have been used to control the release of compounds providing a sustained delivery. The specific product development is the subject of a separate publication and a United States patent application.

This study was designed to determine the in vivo antioxidant activity, using standardized tests, of different amounts of Ambrotose AO® as determined directly by serum $ORAC_{\beta-PE}$ and indirectly by urine lipid hydroperoxide, alkenal and 8-OHdG analyses. Prior and Cao recently suggested that a battery of tests, rather than any one single test, is necessary because some pathological conditions, such as renal failure, can alter any one test quite unrelated to oxidative stress.[22]

Informed consent was obtained from all participants. Twelve healthy male and female adult volunteers taking no nutritional supplements or any drug that would interfere with the study were enrolled. The study subjects, four males and eight females ages 22-61, consumed increasing amounts of the antioxidant supplement. To evaluate $ORAC_{\beta-PE}$ variability over time and to test the reproducibility of the laboratory results, a blood and urine sample was collected from an additional subject who was taking no supplements. During the study, participants continued their normal pre-study daily routines and diets.

Table 5 provides information about the study subjects.

| Table 5. Characteristics of Human Volunteers | | | |
|---|---|---|---|
| **Subject** | **Gender** | **Age** | **Tobacco User** |
| 1 | F | 33 | Yes |
| 2 | M | 41 | No |
| 3 | F | 34 | No |
| 4 | F | 61 | Yes |
| 5 | F | 44 | Yes |
| 6 | M | 22 | No |
| 7 | F | 36 | No |
| 8 | M | 23 | No |
| 9 | F | 38 | Yes |
| 10 | F | 56 | No |
| 11 | F | 53 | No |
| 12 | M | 31 | Yes |

The twelve study subjects had morning fasting serum $ORAC_{\beta-PE}$ and urine analyses performed after an initial washout period of 2 weeks on no supplements and at the end of 2 weeks on each increasing amount of the antioxidant supplement. The amounts used were 500 mg (1 capsule) each day for the first 14 days of supplement use (Period 1), 1.0g (2 capsules) each day for the second 14-day period (Period 2), and 1.5 g (3 capsules) each day during the third 14-day period (Period 3). Additionally, a blood and urine sample from the individual not consuming supplements was analyzed in triplicate to test the precision of the analyses. Blood and urine samples were collected by the independent phlebotomist, immediately packed in dry ice and transported to a local hospital laboratory for preparation. Prepared serum and urine samples were then shipped in dry ice overnight to Genox for independent analysis per the Genox protocol.[23] All samples were then stored at Genox in dry ice, and $ORAC_{\beta-PE}$ and urine measurements were all done at the same time at the conclusion of the study to minimize any analytical reagent variability.

Results: ORAC Values and Percent Change from Baseline Data. The $ORAC_{\beta-PE}$ values and percentage change from baseline for the twelve participants taking Ambrotose AO™ are given in Table 6. Baseline is after the 2-week washout, Period 1 after 2 weeks on 500 mg, Period 2 after 2 weeks on 1.0 g and Period 3 after 2 weeks on 1.5 g. Serum vial labels of some Period 2 samples became detached during shipping to Genox. $ORAC_{\beta-PE}$ values could only be specifically assigned to three of the study participants for this period and not for the rest of the study participants or the additional non-study individual.

Table 2

| | ORAC$_{\beta-PE}$ Values and Percent Change from Baseline | | | | | | |
|---|---|---|---|---|---|---|---|
| | ORAC | | | | % Change from Baseline | | |
| Subject | Baseline | Period 1 | Period 2* | Period 3 | Period 1 | Period 2 | Period 3 |
| 1 | 3300.3 | 5709.4 | | 3117.8 | 73.0% | | -5.5% |
| 2 | 3754.5 | 4618.6 | 6731.8 | 6189.0 | 23.0% | 79.3% | 64.8% |
| 3 | 3695.0 | 3867.9 | | 3995.7 | 4.7% | | 8.1% |
| 4 | 3954.3 | 3199.1 | | 2703.8 | -19.1% | | -31.6% |
| 5 | 3107.3 | 3295.2 | | 4476.2 | 6.0% | | 44.1% |
| 6 | 3313.0 | 5073.8 | | 3156.6 | 53.1% | | -4.7% |
| 7 | 3785.5 | 5069.9 | | 4390.4 | 33.9% | | 16.0% |
| 8 | 3341.2 | 4207.7 | | 4003.2 | 25.9% | | 19.8% |
| 9 | 7568.9 | 6053.2 | 8977.6 | 7734.1 | -20.0% | 18.6% | 2.2% |
| 10 | 4271.8 | 6132.5 | | 6765.0 | 43.6% | | 58.4% |
| 11 | 5619.2 | 6527.0 | 6420.0 | 6844.3 | 16.2% | 14.3% | 21.8% |
| 12 | 5642.9 | 5025.4 | | 4398.7 | -10.9% | | -22.0% |

Statistical Analysis of Raw ORAC Data. A repeated-measures ANOVA was conducted to determine if there was a difference among the Baseline, Period 1, Period 2, and Period 3 raw ORAC data. There were significant differences among the time periods overall ($F(3,24) = 4.02$, $p = .020$). The post hoc analyses revealed that Period 2 was significantly different from Baseline ($t(24) = -3.47$, $p = .002$). Period 1 was significantly different from Period 2 ($t(24) = -2.77$, $p = .011$). Period 2 was significantly different from Period 3 ($t(24) = 2.87$, $p = .009$). The mean for each time period is shown in Table 6.

| Table 6. Mean ORACbeta-PE Values for Each Time Period | | | | |
|---|---|---|---|---|
| | Time Period | Number of Subjects | Mean ORAC Score | ± |
| Baseline | 12 | 4279.5 | 847.9 | |
| Period 1 | 12 | 4898.3 | 699.1 | |
| Period 2 | 3 | 7376.5 | 3466.6 | |
| Period 3 | 12 | 4814.6 | 1053.1 | |

Statistical Analysis of Percent Change from Baseline ORAC Data. In contrast to the raw data used in the analysis reported above (Table 6), this analysis examined differences among the time periods expressed as percent change from baseline. A' repeated-measures ANOVA was conducted to assess differences among the percent change from Baseline of Period 1, Period 2, and Period 3 ORAC data. The omnibus test yielded no significance overall ($F_{(2,13)}$ = 0.71, p = .510). Additionally, the *post hoc* analyses revealed no significant differences among the periods. The mean for each time period is shown in Table 7, with the corresponding boxplots shown in Figure B.

| Table 7. Boxplots for the ORACbeta-PE Percent Change from Baseline by Time Period | | | |
|---|---|---|---|
| Time Period | Number of Subjects | Mean of Percent Change ORAC Score | $\pm$ |
| Period 1 | 12 | 19.1% | 18.1% |
| Period 2 | 3 | 73.4% | 90.3% |
| Period 3 | 12 | 14.3% | 19.0% |

The average lipid hydroperoxide, total alkenal and 8-OHdG values are summarized in Table 8. They are corrected for urine concentration variability by dividing by urine creatinine as measured at the same time on the same sample.

| Table 8. Average Urine Biomarkers for Each 2 Week Time Period | | | | |
|---|---|---|---|---|
| Time Period | Number of Subjects | Hydroperoxide/ creatinine $\mu$M/ (mg/dl) | Alkenal/creatinine $\mu$M/(mg/dl) | 8-OHdG/creatinine (mg/ml)/(mg/dl) |
| Baseline | 12 | 0.0920 | 0.0761 | 0.0724 |
| Period 1 | 12 | 0.0808 | 0.0808 | 0.0861 |
| Period 2 | 12 | 0.0782 | 0.0835 | 0.0740 |
| Period 3 | 12 | 0.0764 | 0.0806 | 0.1025 |

Air Quality. Air quality is known to affect levels of oxidative stress. Increasing concentrations of common pollutants, such as ozone and nitrogen dioxide, decrease air quality. This leads to a greater potential for the generation of ROS.[24,25]. A summary of the average U.S. Environmental Protection Agency (EPA) air quality indexes for each two-week period in the Dallas/Fort Worth (DFW) area, where the subjects lived, is given in Table 6. The EPA uses color codes for area air quality maps: Green: "Good" (0 - 79 parts per billion [ppb] ozone), Yellow: "Moderate" (80 - 99 ppb), Orange: "Unhealthy for Sensitive Groups" (100 - 124 ppb), Red: "Unhealthy"(125 - 149 ppb) and Purple: "Very Unhealthy" (greater than 150 ppb). A numerical value for each daily EPA map of the study area was calculated by assigning numbers to the colors: Green: 1, Yellow: 2, Orange: 3, Red: 4 and Purple: 5 and estimating the numerical average for each day based on the area of each color on the published EPA maps. The daily numerical values were then averaged for each two-week period of the study (Table 9).

| Table 9. Average DFW EPA Air Quality Index for Each Time Period | |
|---|---|
| Time Period | Average EPA Air Quality Index |
| Baseline | 1.0 (range 1.0-1.0) |
| Period 1 | 1.0 (range 1.0-1.1) |
| Period 2 | 1.4 (range 1.0-2.5) |
| Period 3 | 1.8 (range 1.0-2.5) |

Allowing for the uncertainty in assigning values to the study participants in Period 2 as outlined above, the lowest possible average ORAC$_{\beta-PE}$ for the 12 study participants was calculated. Assuming that the lowest 9 values from the serum samples whose labels could not be identified belonged to the participants and combining them with the 3 known values yields the lowest possible average and thus the most conservative estimate of change from baseline. The 9 lowest ORAC$_{\beta-PE}$ values were, 4727.9, 5233.9, 5104.3, 4599.9, 5699.9, 5364.8, 3987.3, 4179.7 and 4584.9. When combined with the 3 known Period 2 values from Table 5, this gave an average ORAC$_{\beta-PE}$ value of 5467.70 for the 12 study participants in Period 2. This is 27.8% above the baseline average of 4279.49.

Discussion. Studies have shown that the consumption of diets rich in fruits and vegetables is protective against oxidative stress.[7,26,17] Dietary guidelines advocate the daily consumption of 2-4 servings of fruit and 3-5 servings of

vegetables.[28] Despite this knowledge and these recommendations, very few individuals in the U.S. consume the recommended daily amounts.

In a U.S. Department of Agriculture (USDA) sponsored clinical study, researchers found that increased consumption of fruits and vegetables, specifically from the usual five to an experimental ten servings a day, significantly increased serum $ORAC_{\beta-PE}$ values by up to approximately 13% after two weeks.[7] In the current study, the increase in average serum $ORAC_{\beta-PE}$ values using each amount of supplement were: 19.1% with 500 mg, 37.4% with 1.0 g, and 14.3% with 1.5 g. These data suggest that the optimal amount that results in the greatest rise in serum $ORAC_{\beta-PE}$ over baseline in healthy people is 1.0 g. The conservative estimate of a 27.8% percent increase with 1.0 g Ambrotose AO™ is more than twice that the published 13% reported individuals consuming five additional fruits and vegetables daily.

The urine lipid hydroperoxide/creatinine values decreased with increasing supplement use. The decrease for each amount was 12.1% with 500 mg, 15.0% with 1.0 g, and 17.0% with 1.5 g, suggesting that protection from lipid damage increased with increasing amounts of supplement over the range studied.

It is unclear why the lipid hydroperoxide data do not correlate exactly with the $ORAC_{\beta-PE}$ values. Serum $ORAC_{\beta-PE}$ is a measure of the antioxidant protection of the blood in regard to its ability to quench free radicals at the time of the measurement. Urine lipid hydroperoxides are a marker of lipid oxidative damage at some time in the past. The temporal relationship between the actual lipid damage and the appearance of lipid hydroperoxides in the urine is not well defined. It may well be that these temporal differences account, in part, for this variance.

In addition to urine lipid hydroperoxides, urine 8-OHdG and alkenals were also measured at each time period. No significant differences nor trends were observed. This again may relate to the temporal relationship between actual lipid and DNA damage and the appearance of the biomarkers in the urine.

Participants in the study lived in the DFW area. The published EPA daily air quality assessments for DFW were averaged for each 2-week period. Air quality was getting worse over the course of the study. This would normally be expected to increase oxidative stress by increasing ROS and hence increasing biomarkers of oxidative damage, such as lipid hydroperoxides.[29] On the contrary, increased protection was evident as measured by increased serum ORAC values. In addition, a downward trend in urine lipid hydroperoxide values and the stability of urine 8-OHdG and alkenals provides evidence of decreased oxidative damage. Taken together, these data suggest that the antioxidant effects afforded by the supplement could have been greater than the effects that were actually measured.

Conclusions. While it is recommended that individuals consume fruits and vegetables according to published guidelines, the reality is that the vast majority will not. These preliminary data suggest that a nutritional supplement containing optimal antioxidant ingredients that preserve antioxidant activity in the finished product increases antioxidant protection in healthy individuals as measured by serum $ORAC_{\beta-PE}$ and decreases lipid oxidative damage as measured by urine lipid hydroperoxides. The protection in healthy people as demonstrated by the increase in serum $ORAC_{\beta-PE}$ over baseline after two weeks was greater using 500 mg, 1.0 g and 1.5 g of Ambrotose AO™ than that documented in published data with the addition of five fruits and vegetables to the diet for two weeks (19.1%, 37.4% and 14.3% vs. 13%).

To our knowledge, this is the first study with an antioxidant supplement to examine four measures of oxidative stress in healthy people and show such an increase in serum $ORAC_{\beta-PE}$. While the optimal range among healthy subjects as suggested by $ORAC_{\beta-PE}$ values was 1 g per day, research has shown that individuals with low serum ORAC values may benefit from high-dose antioxidant supplementation.[30] Hence, those who suffer from increased oxidative stress or otherwise stressed individuals may benefit from larger doses.

While the $ORAC_o$ was used in the antioxidant blend formulation, an independent laboratory used an established method, the $ORAC_{\beta-PE}$, to analyze human clinical trial plasma samples. *In vivo* assessment of antioxidant effectiveness of the Ambrotose AO™ product did not rely on the use of the $ORAC_o$ method.

These data demonstrate that the present antioxidant supplement increases antioxidant protection in consumers as measured by serum ORAC(β-PE) and decreases lipid oxidative damage as measured by urine lipid hydroperoxides.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein.

The scope of the invention is defined by the appended claims.

REFERENCE LIST

1. Koepke CM, Le L, McAnalley S, et al. Results of clinical trials with antioxidants: a review. GlycoScience &Nutrition (Official Publication of GlycoScience corn: The Nutrition Science Site). 2003;4(3): 1-7.

2. Ochi HI Cheng RZ, Kantha SS, et al. The JaICA-genox oxidative stress profile-an overview on the profiling technique in the oxidative stress assessment and management. Biofactors. 2000;13(1-4):195-203.

3. Vivekananthan DP, Penn MS, Sapp SKI et al. Use of antioxidant vitamins for the prevention of cardiovascular disease: meta-analysis of randomised trials. Lancet. 2003;361(9374):2017-2023.

4. Morris CD, Carson S. Routine vitamin supplementation to prevent cardiovascular disease: a summary of the evidence for the U.S. Preventive Services Task Force. Ann Intern Med. 2003;139(1):56-70.

5. Boileau TW, Liao Z, Kim S, et al. Prostate carcinogenesis in N-methyl-N-nitrosourea (NMU)-testosterone-treated rats fed tomato powder, lycopene, or energy-restricted diets. JNatl Cancer Inst. 2003;95(21):1578-1586.

6. Ramberg J, Le L, Vennum E, et al. Why are natural source dietary supplements best? A review based on the vitamin C literature. GlycoScience & Nutrition (Official Publication of GlycoScience com: The Nutrition Science Site). 2003;4(4):1-8.

7. Cao GI Booth SL, Sadowski JA, et al. Increases in human plasma antioxidant capacity after consumption of controlled diets high in fruit and vegetables. Am J Clin Nutr. 1998;68(5):1081-1087.

8. Barhoumi R, Burghardt RG, Busbee DL, et al. Enhancement of glutathione levels and protection from chemically initiated glutathione depletion in rat liver cells by glyconutritionals. Proc Fisher Inst Med Res. 1997;1(1):12-16.

9. Goux WJ, Boyd S, Tone CM, et al. Effect of glyconutritionals on oxidative stress in human subjects: a pilot study. GlycoScience & Nutrition (Official Publication of GlycoScience com: The Nutrition Science Site). 2001;2(12): 1-10.

10. Leonard SS, Cutler D1 Ding MI et al. Antioxidant properties of fruit and vegetable juices: more to the story than ascorbic acid. Ann Clin Lab Sci. 2002;32(2):193-200.

11. Rice-Evans CAI Miller NJ. Antioxidant activities of flavonoids as bioactive components of food. Biochem Soc Trans. 1996;24(3):790-795.

12. Gil MI, Ferreres F, Tomas-Barberan FA. Effect of postharvest storage and processing on the antioxidant constituents (flavonoids and vitamin C) of fresh-cut spinach. JAgric Food Chem. 1999;47(6):2213-2217.

13. Kurilich AC, Jeffery EH, Juvik JA, et al. Antioxidant capacity of different broccoli (Brassica oleracea) genotypes using the oxygen radical absorbance capacity (ORAC) assay. JAgric Food Chem. 2002;50(18):5053-5057.

14. Wang SY, Zheng W, Galletta GJ. Cultural system affects fruit quality and antioxidant capacity in strawberries. JAgric Food Chem. 2002;50(22):6534-6542.

15. Brand JC, Cherikoff V, Lee A, et al. An outstanding food source of vitamin C. Lancet. 1982;2(8303):873.

16. Ramberg J. Green Tea. GlycoScience & Nutrition (Official Publication of GlycoScience com: The Nutrition Science Site). 2003;4(5):1-9.

17. McAnalley S, Koepke C, Lam L, et al. In vitro methods for testing antioxidant potential: a review. GlycoScience & Nutrition (Official Publication of GlycoScience com: The Nutrition Science Site). 2003;4(2): 1-9.

18. Cao GI Alessio HM, Cutler RG. Oxygen-radical absorbance capacity assay for antioxidants [see comments]. Free Radic Biol Med. 1993;14(3):303-311.

19. Esterbauer HI Jurgens GI Quehenberger 0, et al. Autooxidation of human low density lipoprotein: loss of polyunsaturated fatty acids and vitamin E and generation of aldehydes. J Lipid Res. 1987;28(5):495-509.

20. Ohishi N, Ohkawa HI Miike A, et al. A new assay method for lipid peroxides using a methylene blue derivative. Biochem Int. 1985;10(2):205-211.

21. Shigenaga MK, Ames BN. Assays for 8-hydroxy-2'-deoxyguanosine: a biomarker of in vivo oxidative DNA damage. Free Radic Biol Med. 1991;10(3-4):211-216.

22. Prior RL, Cao G. In vivo total antioxidant capacity: Comparison of different analytical methods. Free Radic Biol Med 1999;27((11/12)):1173-1181.

23. Genox Corporation. Oxygen Radical Absorption Capacity Assay for measuring antioxidant activity. October 2001.

24. Rahman I, MacNee W. Role of oxidants/antioxidants in smoking-induced lung diseases. Free Radic Biol Med 1996;21(5):669-681.

25. Cross CE, van der Vliet A, O'Neill CAl et al. Reactive oxygen species and the lung. Lancet. 1994;344(8927): 930-933.

26. Steinmetz KA, Potter JD. Vegetables, fruit, and cancer prevention: a review. J Am Diet Assoc. 1996;96(10): 1027-109.

27. Byers TI Guerrero N. Epidemiologic evidence for vitamin C and vitamin E in cancer prevention. Am J Clin Nutr 1995;62(6 Suppl):1385S-1392s.

28. Nutrition and Your Health: Dietary Guidelines for Americans. Home and Garden Bulletin No. 232, USDA and USDHHS: 1995.

29. Halliwell B, Gutteridge JMC. Free Radicals in Biology and Medicine. 3rd Edition. New York: Oxford University Press, 2000.

30. Schmidt MC, Askew EW, Roberts DE, et al. Oxidative stress in humans training in a cold, moderate altitude environment and their response to a phytochemical antioxidant supplement. Wilderness Environ Med. 2002;13(2): 94-105.

**Claims**

1. An apparatus (10) for detecting directly the antioxidant activity of both lipophilic and lipophobic anti-oxidants comprising:

   a dissolved oxygen sensor (18) in fluid communication with a sample (24) and an oxygen radical sensitive molecule in an organic solvent/water/surfactant mixture having a volume ratio of 1:1:1, wherein the organic solvent is acetone and the surfactant is a non-ionic detergent;
   wherein the oxygen sensor of the apparatus serves to concurrently detect the anti-oxidant effect of both lipophilic and lipophobic anti-oxidants in the organic solvent/water/surfactant mixture by detecting dissolved oxygen in the sample.

2. The apparatus of claim 1, wherein the oxygen radical sensitive molecule is selected from molecules that react with oxygen, molecules with conjugated double bonds; Nitrogen or Sulfur containing compounds, fluorescein, $\beta$ -PE, glutathione-S-transferase, linoleic acid or combinations thereof.

3. The apparatus of claim 1, wherein the dissolved oxygen sensor is a dissolved oxygen meter.

4. The apparatus of claim 1, wherein the dissolved oxygen sensor (18) comprises an electrochemical, a chemiluminescent, a surface plasmon resonance, an infrared, a capacitance coupled, a dye-coupled fiber optic or a hyperspectral oxygen sensor.

5. The apparatus of claim 1, wherein the surfactant comprises Tween.

6. A method of determining anti-oxidant activity comprising the step of:

   determining the dissolved oxygen level in a test solution dissolved in an organic solvent/water/surfactant mixture in the presence of one or more anti-oxidants and an oxygen radical sensitive molecule;
   wherein the anti-oxidant activity of both lipophilic and lipophobic anti-oxidants in the organic solvent/water/surfactant mixture is measured using the dissolved oxygen level determined by a dissolved oxygen detector;

wherein the organic solvent/water/surfactant mixture volume ratio is 1:1:1, the organic solvent is acetone and the surfactant is a non-ionic detergent.

7. The method of claim 6, wherein the dissolved oxygen level is determined using an oxygen detector comprising an electrochemical, chemiluminescent, surface plasmon resonance, capacitance coupled, a dye-coupled fiber optic or a hyperspectral oxygen sensor.

8. The method of claim 6, wherein the anti-oxidant activity of the anti-oxidants is determined using the formula:

$$ORAC(o) = \frac{\dfrac{AUC_{SMP}-AUC_{BLNK}}{AUC_{TRLX}-AUC_{BLNK}} \times 1000\,(mg/gr) \times [TRLX\,(\mu mol/ml)]}{[SMP\,(mg/ml)]}$$

wherein:

AUCsmp is the area under the curve value of the sample, the curve being a plot of oxygen concentration vs. time;
AUCblnk is the area under the curve value of the blank, the curve being a plot of oxygen concentration vs. time;
AUCtrlx is the area under the curve value for 6-hydroxy-2,5,7,8-tetramethylchroman-2-carboxylic acid, the curve being a plot of oxygen concentration vs. time;
TRLX is 6-hydroxy-2,5,7,8-tetramethylchroman-2-carboxylic acid;
SMP is the sample; and
the ORAC(o) value is expressed as the equivalence of micromoles of 6-hydroxy-2,5,7,8-tetramethylchroman-2-carboxylic acid per gram of the sample.

9. The method of claim 6, wherein the surfactant comprises Tween.

10. The method of claim 6, wherein the anti-oxidant activity is measured at about 37 degrees Centrigrade.

11. The method of claim 6, further comprising the step of providing a radical intiator selected from the group consisting of 2,2'-azobis[2-(5-methyl-2-imidazolin-2-yl)propane]dihydrochloride, 2,2' azobis (2-amidinopropane)dihydrochloride (AAPH), 2,2'-azobis(2-amidinopropane)[2-(N-stearyl)amidinopropane] dihydrochloride (SA-1), 2,2'-azo(2-(2-imidiazolin-2-yl)-propane)-[2-[2-(4-n-octyl)imidazolin-2-yl]-propane] dihydrochloride (C-8), 2,2'-azobis(4-methoxy-2,4-dimethylvaleronitrile) (MeO-AMVN), 2,2'-azobis(2,4-dimethylvaleronitrile) (AMVN), azo-bis-isobutylnitrile, 2,2'-azobis (2-methylproprionate) (DAMP), and 2,2'-azobis-(2-amidinopropane), salts or mixtures thereof.

12. The method of claim 6, wherein the detector is disposable.

**Patentansprüche**

1. Vorrichtung (10) zum direkten Detektieren der Antioxidants-Aktivität von sowohl lipophilen als auch lipophoben Antioxidantien, umfassend:

einen Sensor (18) für gelösten Sauerstoff in Fluidverbindung mit einer Probe (24) und einem Sauerstoffradikal-sensitiven Molekül in einer organisches Lösungsmittel/Wasser/Tensid-Mischung mit einem Volumenverhältnis 1:1:1, worin das organische Lösungsmittel Aceton ist und das Tensid ein nichtionisches Detergens ist; worin der Sauerstoffsensor der Vorrichtung dazu dient, gleichzeitig die Antioxidantswirkung von sowohl lipophilen als auch lipophoben Antioxidantien in der organisches Lösungsmittel/Wasser/Tensid-Mischung durch Detektieren von gelöstem Sauerstoff in der Probe zu detektieren.

2. Vorrichtung gemäß Anspruch 1, worin das Sauerstoffradikal-sensitive Molekül ausgewählt ist aus Molekülen, die mit Sauerstoff reagieren, Molekülen mit konjugierten Doppelbindungen; Stickstoff- oder Schwefelhaltigen Verbindungen, Fluorescein, β-PE, Glutathion-S-Transferase, Linolsäure oder Kombinationen hiervon.

3. Vorrichtung gemäß Anspruch 1, worin der Sensor für gelösten Sauerstoff ein Messgerät für gelösten Sauerstoff ist.

4. Vorrichtung gemäß Anspruch 1, worin der Sensor (18) für gelösten Sauerstoff einen elektrochemischen, einen chemilumineszenten, eine Oberflächenplasmaresonanz-, einen Infrarot-, einen kapazitätsgekoppelten, einen farbstoffgekoppelten faseroptischen oder einen hyperspektralen Sauerstoffsensor umfasst.

5. Vorrichtung gemäß Anspruch 1, worin das Tensid Tween umfasst.

6. Verfahren zum Bestimmen der Antioxidants-Aktivität, umfassend die Schritte:

Bestimmen des gelösten Sauerstofflevels in einer Testlösung, die in einer organisches Lösungsmittel/Wasser/Tensid-Mischung gelöst ist, in der Gegenwart von einem oder mehr Antioxidantien und einem Sauerstoffradikal-sensitiven Molekül;
worin die Antioxidants-Aktivität von sowohl lipophilen als auch lipophilen Anti-Oxidanzien in der organisches Lösungsmittel/Wasser/Tensid-Mischung unter Verwendung des Levels von gelöstem Sauerstoff, bestimmt mit einem Detektor für gelösten Sauerstoff, gemessen wird;
worin das organische Lösungsmittel/Wasser/Tensid-Mischungsvolumenverhältnis 1:1:1 beträgt, das organische Lösungsmittel Aceton ist und das Tensid ein nichtionisches Detergens ist.

7. Verfahren gemäß Anspruch 6, worin der gelöste Sauerstofflevel unter Verwendung eines Sauerstoffdetektors bestimmt wird, der einen elektrochemischen, chemilumineszenten, Oberflächenplasmaresonanz-, kapazitätsgekoppelten, farbstoffgekoppelten faseroptischen oder hyperspektralen Sauerstoffsensor umfasst.

8. Verfahren gemäß Anspruch 6, worin die Antioxidants-Aktivität des Antioxidants unter Verwendung der Formel:

$$ORAC(o) = \frac{\dfrac{AUC_{SMP} - AUC_{BLNK}}{AUC_{TRLX} - AUC_{BLNK}} \times 1000\ (mg/gr) \times [TRLX\ (\mu mol/mol)]}{[SMP\ (mg/ml)]}$$

bestimmt wird, worin:

AUCsmp die Fläche unter dem Kurvenwert der Probe ist, worin die Kurve eine Auftragung der Sauerstoffkonzentration gegen die Zeit ist;
AUCblnk die Fläche unter dem Kurvenwert der Leerprobe ist, wobei die Kurve eine Auftragung der Sauerstoffkonzentration gegen die Zeit ist;
AUCtrlx die Fläche unter dem Kurvenwert für 6-Hydroxy-2,5,7,8-tetramethylchroman-2-carbonsäure ist, wobei die Kurve eine Auftragung der Sauerstoffkonzentration gegen die Zeit ist;
TRLX 6-Hydroxy-2,5,7,8-tetramethylchroman-2-carbonsäure ist;
SMP die Probe ist; und
der ORAC(o)-Wert ausgedrückt ist als die Äquivalenz von Mikromolen von 6-Hydroxy-2,5,7,8-tetramethylchroman-2-carbonsäure je Gramm der Probe.

9. Verfahren gemäß Anspruch 6, worin das Tensid Tween umfasst.

10. Verfahren gemäß Anspruch 6, worin die Antioxidants-Aktivität bei etwa 37°C gemessen wird.

11. Verfahren gemäß Anspruch 6, ferner umfassend einen Schritt, worin ein Radikalinitiator bereitgestellt wird, der ausgewählt aus der Gruppe bestehend aus 2,2'Azobis[2-(5-methyl-2-imidazolin-2-yl)propan]dihydrochlorid, 2,2'-(Azobis(2-amidinopropan)dihydrochlorid (AAPH), 2,2'-Azobis(2-amidinopropan)[2-(N-stearyl)amidinopropan]dihydrochlorid (SA-1), 2,2'-Azo(2-(2-imidiazolin-2-yl)propan)-[2-[2-(4-n-octyl)imidazolin-2-yl]propan]dihydrochlorid (C-8), 2,2'-Azobis(4-methoxy-2,4-dimethylvaleronitril) (MeO-AMVN), 2,2'-Azobis(2,4-dimethylvaleronitril) (AMVN), Azo-bis-isobutylnitril, 2,2'-Azobis(2-methylpropionat) (DAMP) und 2,2'-Azobis-(2-amidinopropan), Salzen oder Mischungen hiervon.

**12.** Verfahren gemäß Anspruch 6, worin der Detektor ein Einwegartikel ist.

**Revendications**

**1.** Appareil (10) permettant de détecter directement l'activité antioxydante à la fois d'antioxydants lipophiles et lipophobes comprenant :

un capteur d'oxygène dissous (18) en communication fluidique avec un échantillon (24) et une molécule sensible aux radicaux oxygène dans un mélange solvant organique/eau/tensioactif ayant un rapport en volume de 1:1:1, dans lequel le solvant organique est l'acétone et le tensioactif est un détergent non ionique ;
dans lequel le capteur d'oxygène de l'appareil sert à détecter simultanément l'effet antioxydant à la fois d'antioxydants lipophiles et lipophobes dans le mélange solvant organique/eau/tensioactif en détectant l'oxygène dissous dans l'échantillon.

**2.** Appareil selon la revendication 1, dans lequel la molécule sensible aux radicaux oxygène est choisie parmi les molécules qui réagissent avec l'oxygène, les molécules avec des doubles liaisons conjuguées ; les composés contenant de l'azote ou du soufre, la fluorescéine, le $\beta$-PE la glutathione-S-transférase, l'acide linoléique ou leurs combinaisons.

**3.** Appareil selon la revendication 1, dans lequel le capteur d'oxygène dissous est un appareil de mesure d'oxygène dissous.

**4.** Appareil selon la revendication 1, dans lequel le capteur d'oxygène dissous (18) comprend un capteur d'oxygène électrochimique, chimioluminescent, à résonance plasmonique de surface, infrarouge, couplée à une capacitance, à fibre optique couplée à un colorant ou hyperspectral.

**5.** Appareil selon la revendication 1, dans lequel le tensioactif comprend du Tween.

**6.** Procédé de détermination de l'activité antioxydante comprenant l'étape de :

détermination du taux d'oxygène dissous dans une solution d'essai dissoute dans un mélange solvant organique/eau/tensioactif en présence d'un ou plusieurs antioxydants et d'une molécule sensible aux radicaux oxygène ;
dans lequel l'activité antioxydante à la fois d'antioxydants lipophiles et lipophobes dans le mélange solvant organique/eau/tensioactif est mesurée en utilisant le taux d'oxygène dissous déterminé par un détecteur d'oxygène dissous ;
dans lequel le rapport en volume du mélange solvant organique/eau/tensioactif est de 1:1:1, le solvant organique est l'acétone et le tensioactif est un détergent non ionique.

**7.** Procédé selon la revendication 6, dans lequel le taux d'oxygène dissous est déterminé en utilisant un détecteur d'oxygène comprenant un capteur d'oxygène électrochimique, chimioluminescent, à résonance plasmonique de surface, infrarouge, couplée à une capacitance, à fibre optique couplée à un colorant ou hyperspectral.

**8.** Procédé selon la revendication 6, dans lequel l'activité antioxydante des antioxydants est déterminée à l'aide de la formule :

$$ORAC(o) = \frac{\frac{ASC_{SMP} - ASC_{BLNK}}{ASC_{TRLX} - ASC_{BLNK}} \times 1\,000\ (mg/g) \times [TRLX\ (\mu mol/mL)]}{[SMP\ (mg/mL)]}$$

dans laquelle :

ASCsmp est l'aire sous la courbe valeur de l'échantillon, la courbe étant un graphique de la concentration en oxygène en fonction du temps ;
ASCblnk est l'aire sous la courbe valeur du blanc, la courbe étant un graphique de la concentration en oxygène en fonction du temps ;

ASCtrlx est l'aire sous la courbe valeur pour l'acide 6-hydroxy-2,5,7,8-tétraméthylchroman-2-carboxylique, la courbe étant un graphique de la concentration en oxygène en fonction du temps ;

TRLX est l'acide 6-hydroxy-2,5,7,8-tétraméthylchroman-2-carboxylique ;

SMP est l'échantillon ; et

la valeur ORAC(o) est exprimée comme l'équivalence de micromoles d'acide 6-hydroxy-2,5,7,8-tétraméthylchroman-2-carboxylique par gramme de l'échantillon.

9. Procédé selon la revendication 6, dans lequel le tensioactif comprend du Tween.

10. Procédé selon la revendication 6, dans lequel l'activité antioxydante est mesurée à environ 37 degrés Centigrade.

11. Procédé selon la revendication 6, comprenant en outre l'étape de fourniture d'un initiateur de radicaux choisi dans le groupe consistant en 2,2'-azobis[2-(5-méthyl-2-imidazolin-2-yl)propane]dichlorhydrate, 2,2'-azobis(2-amidinopropane)dichlorhydrate (AAPH), 2,2'-azobis(2-amidinopropane)[2-(N-stéaryl)amidinopropane]dichlorhydrate (SA-1), 2,2'-azo(2-(2-imidiazolin-2-yl)-propane)-[2-[2-(4-n-octyl)imidazolin-2-yl]-]propane]dichlorhydrate (C-8), 2,2'-azo-bis(4-méthoxy-2,4-diméthylvaléronitrile) (MeO-AMVN), 2,2'-azobis(2,4-diméthylvaléronitrile) (AMVN), azo-bis-isobutylnitrile, 2,2'-azobis(proprionate de 2-méthyle) (DAMP), et 2,2'-azobis-(2-amidinopropane), leurs sels ou mélanges.

12. Procédé selon la revendication 6, dans lequel le détecteur est jetable.

*Fig. 1*

```
                                                              ⌐50
                                                            ⌡

  ┌──────────────────────────────────────────────┐
  │         EQUILIBRATE OXYGEN PROBE IN           │
  │         SOLVENT: WATER:DETERGENT              │
  │                    52                         │
  └──────────────────────────────────────────────┘
```

| DISSOLVE ANTI-OXIDANT STANDARD IN SOLVENT:WATER:DETERGENT 54 | DISSOLVED SAMPLE IN SOLVENT:WATER:DETERGENT 66 |
|---|---|
| ADD OXYGEN RADICAL TARGET 56 | ADD OXYGEN RADICAL TARGET 68 |
| CHALLENGE ANTI-OXIDANT STANDARD WITH OXYGEN RADICAL GENERATOR IN SOLVENT:WATER:DETERGENT 58 | CHALLENGE ANTI-OXIDANT SAMPLE WITH OXYGEN RADICAL GENERATOR IN SOLVENT:WATER:DETERGENT 70 |
| MEASURE ANTI-OXIDANT STANDARD IN SOLVENT:WATER:DETERGENT 60 | MEASURE SAMPLE IN SOLVENT:WATER:DETERGENT 72 |

```
  ┌──────────────────────────────────────────────┐
  │                  SUBTRACT                      │
  │         SOLVENT: WATER:DETERGENT              │
  │                 BACKGROUND                     │
  │                    62                         │
  └──────────────────────────────────────────────┘

  ┌──────────────────────────────────────────────┐
  │   COMPARE SAMPLE VALUE TO KNOWN ANTI-         │
  │         OXIDANT STANDARD VALUE                 │
  │                    64                         │
  └──────────────────────────────────────────────┘
```

*Fig. 2*

## ORAC$_0$

Percent Oxygen

blank

| Initiator: | AAPH |
| Standard: | Trolox |
| Target Molecule: | Linoleic Acid |

Time

## ORAC$_{fl}$

Fluorescence

| Initiator: | AAPH |
| Standard: | Trolox |
| Target Molecule: | Flourescein |

Time

*Fig. 3*

Fig. 4

Fig. 5

*Fig. 6*

*Fig. 7*

*Fig. 8*

*Fig. 9*

Fig. 10

Fig. 11

*Fig. 12A*

*Fig. 12B*

Fig. 13

Fig. 14

*Fig. 15*

*Fig. 16*

*Fig. 17*

EP 1 664 746 B1

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 64804703 A
- US 5518590 A
- US 6638415 B
- US 20020182736 A
- US 2003072770 A

## Non-patent literature cited in the description

- **GHISELLI A ; SERAFINI M ; MAIANI G ; AZZINI E ; FERRO-LUZZI A.** A fluorescence-based method for measuring total plasma antioxidant capability. *Free Radic. Biol. Med.,* January 1995, vol. 18, 29-36
- **HUANG D et al.** *J. Agric. Food Chem.,* 2002, vol. 50, 1815-1821
- **BERGMAN M et al.** *Phytochemistry,* 2001, vol. 58, 143-152
- **MURRAY et al.** *Harper's Biochemistry,* 1996
- **ZUBAY et al.** Principles of Biochemistry. 1995, vol. II
- **STRYER.** *Stryer's Biochemistry,* 1995
- Merck Index. 1996
- **S. M. BERGE et al.** *J. Pharmaceutical Sciences,* 1977
- **OU, B. ; HAMPSCH-WOODILL, M. ; PRIOR, R.L.** *J. Agric. Food Chem.,* 2001, vol. 49, 4619-4626
- **OU et al.** Development and Validation of Oxygen Radical Absorbance Capacity Assay for Lipophilic Antioxidants Using Randomly Methylated-β-Cyclodextrin as the Solubility Enhancer. *J. Agric. Food Chem.,* 2002, vol. 50, 1815-1821
- **PIZZORNO ; MURRAY.** Textbook of Natural Medicine. Churchill Livingston, 1999
- **LEAF A. ; LAUNOIS J.** A Scientist Visits Some of the World's Oldest People. *National Geographic,* January 1973
- **KOENIG R.** Sardinia's Mysterious Male Methuselahs. *Science,* 16 March 2001
- **KOEPKE CM ; LE L ; MCANALLEY S et al.** Results of clinical trials with antioxidants: a review. *Glyco-Science &Nutrition (Official Publication of Glyco-Science corn: The Nutrition Science Site,* 2003, vol. 4 (3), 1-7
- **OCHI HI ; CHENG RZ ; KANTHA SS et al.** The Ja-ICA-genox oxidative stress profile-an overview on the profiling technique in the oxidative stress assessment and management. *Biofactors,* 2000, vol. 13 (1-4), 195-203
- **VIVEKANANTHAN DP ; PENN MS ; SAPP SKI et al.** Use of antioxidant vitamins for the prevention of cardiovascular disease: meta-analysis of randomised trials. *Lancet,* 2003, vol. 361 (9374), 2017-2023
- **MORRIS CD ; CARSON S.** Routine vitamin supplementation to prevent cardiovascular disease: a summary of the evidence for the U.S. Preventive Services Task Force. *Ann Intern Med.,* 2003, vol. 139 (1), 56-70
- **BOILEAU TW ; LIAO Z ; KIM S et al.** Prostate carcinogenesis in N-methyl-N-nitrosourea (NMU)-testosterone-treated rats fed tomato powder, lycopene, or energy-restricted diets. *JNatl Cancer Inst.,* 2003, vol. 95 (21), 1578-1586
- **RAMBERG J ; LE L ; VENNUM E et al.** Why are natural source dietary supplements best? A review based on the vitamin C literature. *GlycoScience & Nutrition (Official Publication of GlycoScience com: The Nutrition Science Site,* 2003, vol. 4 (4), 1-8
- **CAO GI ; BOOTH SL ; SADOWSKI JA et al.** Increases in human plasma antioxidant capacity after consumption of controlled diets high in fruit and vegetables. *Am J Clin Nutr.,* 1998, vol. 68 (5), 1081-1087
- **BARHOUMI R ; BURGHARDT RG ; BUSBEE DL et al.** Enhancement of glutathione levels and protection from chemically initiated glutathione depletion in rat liver cells by glyconutritionals. *Proc Fisher Inst Med Res.,* 1997, vol. 1 (1), 12-16
- **GOUX WJ ; BOYD S ; TONE CM et al.** Effect of glyconutritionals on oxidative stress in human subjects: a pilot study. *GlycoScience & Nutrition (Official Publication of GlycoScience com: The Nutrition Science Site,* 2001, vol. 2 (12), 1-10
- **LEONARD SS ; CUTLER D1 ; DING MI et al.** Antioxidant properties of fruit and vegetable juices: more to the story than ascorbic acid. *Ann Clin Lab Sci.,* 2002, vol. 32 (2), 193-200

47

- **RICE-EVANS CAI ; MILLER NJ.** Antioxidant activities of flavonoids as bioactive components of food. *Biochem Soc Trans.,* 1996, vol. 24 (3), 790-795
- **GIL MI ; FERRERES F ; TOMAS-BARBERAN FA.** Effect of postharvest storage and processing on the antioxidant constituents (flavonoids and vitamin C) of fresh-cut spinach. *JAgric Food Chem.,* 1999, vol. 47 (6), 2213-2217
- **KURILICH AC ; JEFFERY EH ; JUVIK JA et al.** Antioxidant capacity of different broccoli (Brassica oleracea) genotypes using the oxygen radical absorbance capacity (ORAC) assay. *JAgric Food Chem.,* 2002, vol. 50 (18), 5053-5057
- **WANG SY ; ZHENG W ; GALLETTA GJ.** Cultural system affects fruit quality and antioxidant capacity in strawberries. *JAgric Food Chem.,* 2002, vol. 50 (22), 6534-6542
- **BRAND JC ; CHERIKOFF V ; LEE A et al.** An outstanding food source of vitamin C. *Lancet,* 1982, vol. 2 (8303), 873
- **RAMBERG J.** Green Tea. *GlycoScience & Nutrition (Official Publication of GlycoScience com: The Nutrition Science Site,* 2003, vol. 4 (5), 1-9
- **MCANALLEY S ; KOEPKE C ; LAM L et al.** In vitro methods for testing antioxidant potential: a review. *GlycoScience & Nutrition (Official Publication of GlycoScience com: The Nutrition Science Site,* 2003, vol. 4 (2), 1-9
- **CAO GI ; ALESSIO HM ; CUTLER RG.** Oxygen-radical absorbance capacity assay for antioxidants [see comments. *Free Radic Biol Med.,* 1993, vol. 14 (3), 303-311
- **ESTERBAUER HI ; JURGENS GI ; QUEHEN-BERGER 0 et al.** Autooxidation of human low density lipoprotein: loss of polyunsaturated fatty acids and vitamin E and generation of aldehydes. *J Lipid Res.,* 1987, vol. 28 (5), 495-509
- **OHISHI N ; OHKAWA HI ; MIIKE A et al.** A new assay method for lipid peroxides using a methylene blue derivative. *Biochem Int.,* 1985, vol. 10 (2), 205-211
- **SHIGENAGA MK ; AMES BN.** Assays for 8-hydroxy-2'-deoxyguanosine: a biomarker of in vivo oxidative DNA damage. *Free Radic Biol Med,* 1991, vol. 10 (3-4), 211-216
- **PRIOR RL ; CAO G.** In vivo total antioxidant capacity: Comparison of different analytical methods. *Free Radic Biol Med,* 1999, vol. 27 (11/12), 1173-1181
- Oxygen Radical Absorption Capacity Assay for measuring antioxidant activity. Genox Corporation, October 2001
- **RAHMAN I ; MACNEE W.** Role of oxidants/antioxidants in smoking-induced lung diseases. *Free Radic Biol Med,* 1996, vol. 21 (5), 669-681
- **CROSS CE ; VAN DER VLIET A ; O'NEILL CAI et al.** Reactive oxygen species and the lung. *Lancet,* 1994, vol. 344 (8927), 930-933
- **STEINMETZ KA ; POTTER JD.** Vegetables, fruit, and cancer prevention: a review. *J Am Diet Assoc.,* 1996, vol. 96 (10), 1027-109
- **BYERS TI ; GUERRERO N.** Epidemiologic evidence for vitamin C and vitamin E in cancer prevention. *Am J Clin Nutr,* 1995, vol. 62 (6), 1385S-1392s
- Nutrition and Your Health: Dietary Guidelines for Americans. Home and Garden Bulletin No. 232. USDA and USDHHS, 1995
- **HALLIWELL B ; GUTTERIDGE JMC.** Free Radicals in Biology and Medicine. Oxford University Press, 2000
- **SCHMIDT MC ; ASKEW EW ; ROBERTS DE et al.** Oxidative stress in humans training in a cold, moderate altitude environment and their response to a phytochemical antioxidant supplement. *Wilderness Environ Med.,* 2002, vol. 13 (2), 94-105